# EUROPEAN PATENT APPLICATION

(11) **EP 1 156 116 A1**
(43) Date of publication of application: **21.11.2001**
(21) Application number: 00401402.3
(22) Date of filing: 19.05.2000
(51) Int. Cl.: C12N 15/82, C12N 5/14, C12N 1/15, C12N 1/21, C07K 14/415

(54) **Nucleotide sequences coding for signal transduction components involved in plant pathogen defense**

(71) Applicant: KEYGENE N.V., 6700 AE Wageningen (NL)
(72) Inventor: Haring, Michel Albertus, 2024 RL Haarlem (NL); Vossen, Jacobus Hubertus, 1051 EA Amsterdam (NL); Simons, Augustinus Franciscus Maria, 6715 JL Ede (NL); Cornelissen, Bernardus Johannes Clemens, 2361 EB Warmond (NL)
(74) Representative: Desaix, Anne

(57) **Abstract**

The invention relates to nucleotide sequences derived from a plant genome, encoding a polypeptide product which can be activated as a signaling molecule in the signal transduction pathway of resistance to a plant pathogen which resistance results from the interaction between polypeptide products encoded by a resistance gene and elicitors encoded by an avirulence gene in said soil plant pathogen, wherein the polypeptide product encoded by the nucleotide sequence interacts with a polypeptide product encoded by said resistance gene.

The invention also relates to the use of these sequences in the establishment of durable and broad-range resistance strategies based on plant defence

## Description

The invention relates to identified nucleotide sequence coding for signal transduction components that may be involved in the resistance of plant crops to various pathogens.

The present invention further concerns the products, especially polypeptide products encoded by said nucleotide sequences, and to the use of said nucleotide sequences and encoded products in inducing or contributing to resistance of plants to pathogens and especially to soil plant pathogens.

Disease management in agriculture largely depends on the breeding of cultivars that carry monogenic resistances against a variety of pathogens. Often these resistances are absolute and specific for one race of a pathogen only. This puts a selective pressure on the pathogen that results in the appearance of new races which break the resistance. Breeding for tolerance against pathogen infections would circumvent this selective pressure, but is in practice difficult because such traits are determined by multiple loci.

Using molecular techniques various natural disease resistance genes have been isolated during the last five years. However, their use in molecular breeding programs is limited since they code for resistance to one specific race of a pathogen only. To engineer broad spectrum resistance traits very different strategies are being pursued. The most wide-spread approach used for instance for fungus resistance is the expression of genes encoding proteins inhibiting fungal growth. In the last few years strategies for fungal resistance have been explored based on the induction by pathogens of cell death at the site of infection.

Plant-pathogen interactions result either in an invasion of the plant by the pathogen, causing disease symptoms and eventually the death of the plant, or in an infection localized in a restricted area of plant tissue or even in a few cells. Localization of infection is due to defence reactions of the plant. The defence response best studied thusfar is the race-specific resistance reaction brought about by single, dominant resistance (R-) genes. Genetically, race-specific resistance is explained by the gene-for-gene hypothesis which states that the outcome of a plant-pathogen interaction is determined by the simultaneous presence of both an avirulence (avr) gene in the pathogen and the corresponding R-gene in the host (Flor, 1971). Biochemically, the product of a pathogen avr-gene, i.e., the race-specific elicitor, is thought to be recognized by a specific receptor, i.e., the product of a corresponding R-gene in the plant; upon recognition expression of resistance is induced.

For many interactions the existence of both an R-gene and a corresponding avr-gene has been proven, and sofar more than 20 R-genes have been cloned. In a few cases corresponding pathogen avr-genes have been isolated as well. The primary structures of the products of the cloned R-genes seem to confirm the receptor-like character of these proteins (Staskawicz et al., 1995). In addition, the conserved structure of the proteins encoded by the R-genes suggests that the defence responses, induced upon recognition of a race-specific elicitor, are likely to contain conserved signal transduction components. Sofar, no sequence conservation has been observed in avr-gene products despite their functional similarity.

In an attempt to develop durable resistance in crops, the present invention proposes to identify downstream signaling components of race-specific resistance. The invention therefore provides access to compositions, including nucleotide sequences or polypeptide products that may be used in the activation of defence mechanisms in plants, especially against soil plant pathogens.

Race specific resistance is established by resistance (R-) gene products that recognise pathogen-race derived ligands. The recognition signal is transduced by the R-gene product into a defence response by activating downstream signaling molecules. Recent research suggests that different R-genes utilise identical downstream components (*EDS-1*, *NDR-1* from Arabidopsis). R-gene independent activation of such genes might therefore trigger defence reactions independently of specific pathogen recognition.

In order to identify downstream signaling genes, the inventors have started from a particular resistance gene, i.e., the *I-2* gene from tomato and have identified proteins that physically interact with the resistance gene *I-2* product from tomato. The *I-2* gene confers resistance to the soil borne pathogen *Fusarium oxysporum* f.sp. *lycopersici* (Fol), carrying avirulence gene 2. The protein encoded by the *I-2* gene contains a nucleotide binding site (NBS), a leucine zipper (LZ) domain and a leucine-rich repeat region (LRR). These regions show homologies to similar domains in proteins encoded by other R-genes including *RPM1, RPS2, L6* and many others.

The invention thus relates to a nucleotide sequence derived from a plant genome, encoding a polypeptide product which can be activated as a signaling molecule in the signal transduction pathway of resistance to a plant pathogen wherein said resistance results from the interaction between polypeptide products encoded by a resistance gene and elicitors encoded by an avirulence gene in said plant pathogen.

According to a preferred embodiment of the invention, the above plant pathogen is a soil plant pathogen. Within the group of soil plant pathogens, it is especially referred to fungi, especially Fusarium oxysporum, Verticillium Dahliae, nematodes, bacteria like Ralstona Solanaceum.

The expression "derived from a plant genome" indicates that the nucleotide sequence can be isolated from a plant genome but the expression also encompasses any sequence, whatever its preparation process or its origin, which can be prepared on the basis of the knowledge of the nucleotide sequence and/or of the function of the original nucleotide sequence present in the plant genome. For instance, so-called nucleotide sequences "derived from a plant genome" encompass any nucleotide sequence identical or modified with respect to the originally identified plant sequence, provided its structure and/or activity within the scope of the present invention, is maintained, improved, repressed or modified with respect to the activity of the originally identified plant sequence, for the purpose of the invention.

Therefore, a nucleotide sequence according to the invention can be any type of nucleotide sequence including DNA, RNA and especially can be a genomic or synthetic DNA sequence, for instance a complementary DNA sequence (cDNA). Said sequence can be used in sense or anti-sense orientation for the purpose of the invention.

A nucleotide sequence within the scope of the invention, can have essentially the same length as the original sequence or can be shorter or to the contrary longer. It can be chosen for its capacity to hybridize, in stringent conditions, to the coding part of the original sequence present in the plant genome.

According to the above definition, a nucleotide sequence of the invention is capable of encoding a polypeptide product. This encompasses nucleotide sequences corresponding to the Open Reading Frame capable of being expressed as an aminoacid sequence. It also encompasses the nucleotide sequence provided with necessary or appropriate control elements for the transcription and/or expression of said nucleotide sequence, these sequences for the control including especially promoter sequences and if necessary additional sequences like enhancers and other well-known control sequences. The control of the transcription and/or expression may be directed by homologous sequences or heterologous sequences with respect to the coding sequence.

Therefore, the nucleotide sequence according to the above definition comprises and in a particular embodiment, corresponds to, the nucleotide sequence of the Open Reading Frame (ORF) encoding the aminoacid sequence of the polypeptide product which can be activated in a plant or plant part, as a signaling molecule in the signal transduction pathway of resistance of said plant to plant pathogens, in particular soil plant pathogens, as a result of the interaction between polypeptide products encoded by resistance genes and race-specific elicitors encoded by avirulence genes in said plant pathogens.

According to another embodiment, the nucleotide sequence of the invention is a fragment of the sequence of the ORF and encodes a polypeptide which is a part of the original signaling molecule, provided it has the essential activity of the original signaling molecule relating to the purpose of the invention.

According to the above definitions, the putative function or activity of the polypeptide product encoded by the nucleotide sequence of the invention is disclosed with respect to the function which has been observed for said sequence, in the resistance mechanism to plant pathogens, especially in the signaling pathway when it is expressed in a biological environment that permits the expression of its activity.

The invention is however also directed to a nucleotide sequence defined according to one or several definitions above or hereafter, including any available combination of the preferred embodiments, in a process or in a use that would confer to said sequence a different function or that would enable it to be active in a different biological environment from its natural one. This would apply for instance if the nucleotide sequence or encoded product therefrom is used in a non race-specific defence mechanism against pathogens, in particular plant pathogens.

According to another particular embodiment of the invention, the nucleotide sequence is characterized in that it codes for a polypeptide product that interacts with the N-terminal part or with the C-terminal part of the polypeptide product encoded by the resistance gene to plant pathogens especially to soil plant pathogens.

By the expressions "in N-terminal part" and "C-terminal part", it is understood that the sequences concerned are those of respectively the first 2/3 of the sequence of the polypeptide or the last 1/3 of said polypeptide sequence of reference.

The invention especially relates to a nucleotide sequence derived from a plant genome, which encodes a polypeptide product that interacts with the polypeptide product encoded by the *I-2* resistance gene.

The *I-2* resistance gene has been disclosed in European patent application No. 96929215.0 filed on August 6, 1996.

The inventors have observed as a result of the experiments performed by use of said *I-2* resistance gene that two groups of nucleotide sequences of the invention, also designated interactor sequences, can be identified with respect to their interaction, especially physical interaction, with the I-2 polypeptide product.

Thus, the invention relates to, on the one hand, nucleotide sequences that interact with the N-terminal part of the polypeptide product encoded by the *I-2* resistance gene and on the other hand, to nucleotide sequences encoding products that interact with the C-terminal part of the polypeptide product encoded by the *I-2* resistance gene.

Testing the interaction may be achieved in accordance with the assays described in the following experimental procedures.

In a particular embodiment of the invention, the nucleotide sequence encodes a signal transduction component.

An appropriate method for the identification of nucleotide sequences of the invention may be based on the two-hybrid technology described in the prior art, which method enables the detection of protein-protein interactions. Two-hybrid systems are commercially available for instance by company CLONTECH (Palo-Aldo Ca. USA).

Accordingly, a bait component is constructed that comprises a vector comprising a nucleotide sequence of a resistance gene of a determined plant directing the expression of the polypeptide product, fused to a DNA-binding moiety. A prey component is also prepared that comprises a sequence or a sequence library to be assayed (capable of encoding interactor proteins) which can be prepared from DNA for instance cDNA of a plant susceptible to a determined plant pathogen corresponding to said resistance gene of the bait and recombined with an activation domain that functionally interacts with said binding domain. A third component is provided that comprises one or several reporter genes whose expression is made dependent from the interaction of the bait and prey components involving interaction of the nucleotide sequence of the resistance gene and the assayed nucleotide sequence.

In a particular embodiment of the invention, the resistance gene of interest is the *I-2* gene of tomato or a fragment thereof, especially a functional domain of said gene. It is recombined with the Binding domain of a transcription factor and expressed in a determined yeast strain suitable to carry out the selection of interactor proteins. It constitutes a bait component.

A prey component or a prey library is prepared wherein an appropriate vector is used to clone and express nucleotide sequences capable of encoding polypeptide products that may be interactor candidates to interact with said *I-2* gene, or fragment thereof.

The vector construct of the prey is prepared so that the nucleotide sequences of the library are fused with an activation domain of the transcription factor capable of interacting with the above binding domain.

Said vector is used to express the cloned library in the yeast strain expressing the bait and a screening of interactor proteins is performed to detect yeast transformants expressing marker genes whose expression is under the control of a promoter activated by the interaction between the binding domain of the bait and the activation domain of the prey through interaction of the candidate interactor gene and nucleotide sequence of the resistance gene of the plant.

The invention can, in addition or alternatively, be defined by the fact that the nucleotide sequence can be obtained by the process comprising:
a) cloning into a yeast strain, one or several vectors comprising a construct comprising at least one determined region of a resistance gene to a plant pathogen, encoding a polypeptide product of said resistance gene to a plant pathogen wherein said at least determined region of said resistance gene is fused to a sequence coding for a binding domain of a transcription factor capable of activating a reporter gene present in the vector and placed under the control of regulation sequences whose activation is dependent upon interaction of the binding domain and of the activation domain of the transcription factor,
b) cloning into one or several vectors, a nucleotide sequence or a cDNA library obtained from a plant, which may be susceptible to said plant pathogen or is naturally resistant to said plant pathogen or has been rendered resistant to said plant pathogen, the cloned nucleotide sequences being fused in the vector, with a nucleotide sequence encoding the activation domain of said transcription factor,
c) expressing said vectors of steps a) and b) in said yeast strain,
d) identifying positive clones resulting in activation of the marker gene cloned into the first or second vectors.

Said plant pathogen is advantageously a soil plant pathogen.

In a particular embodiment of said process, the nucleotide sequence, especially the cDNA fragment expressed by the positive clones of yeast is identified and if appropriate isolated.

It can in addition be sequenced according to well-known techniques.

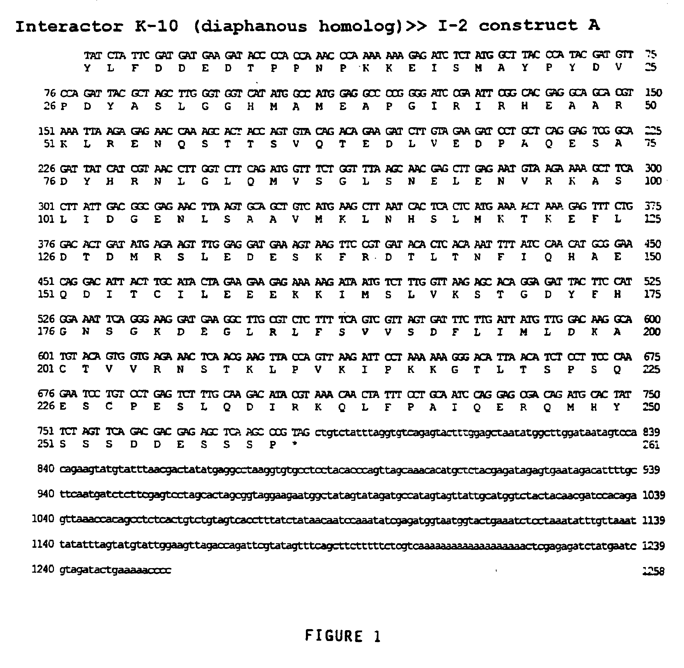

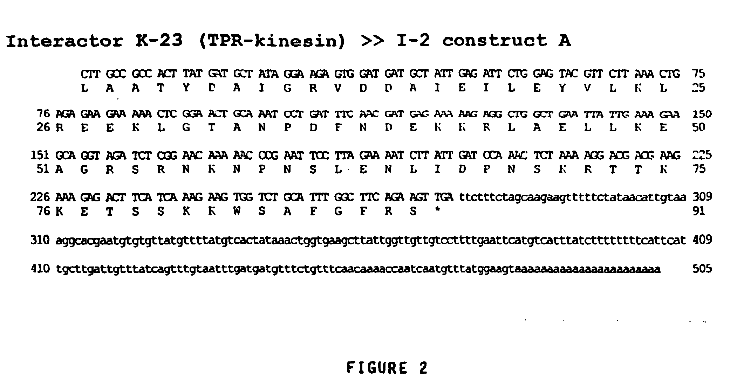

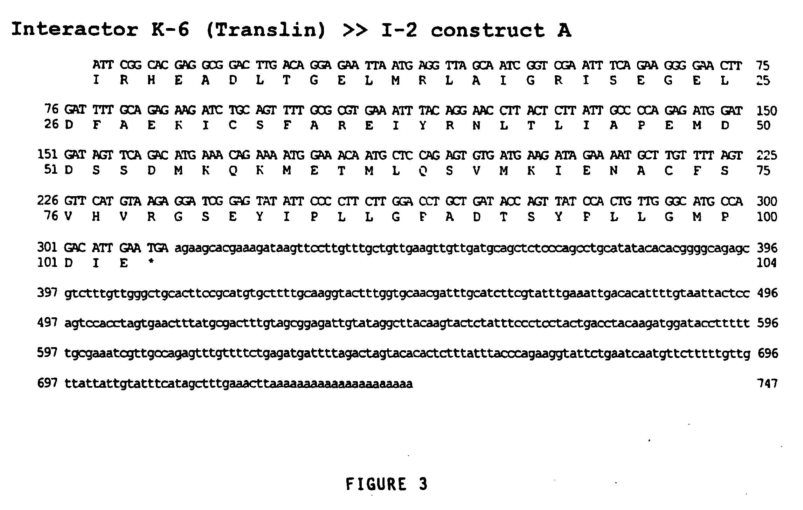

As examples of interactor sequences, the invention relates to those designated by interactor K-10, interactor K-23 and interactor K-6 respectively represented in figures 1 to 3 and having a capacity of interacting with the N-terminal domain of the I-2 polypeptide product.

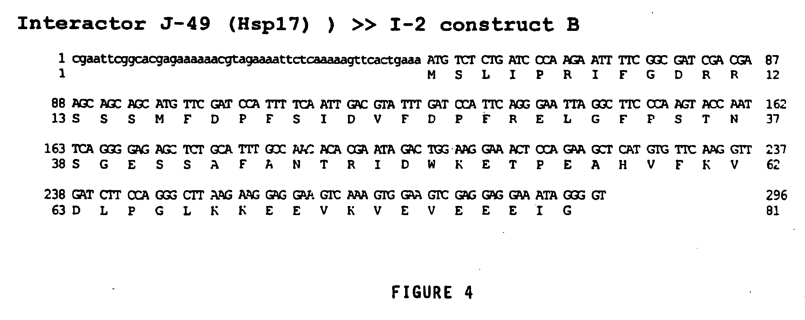

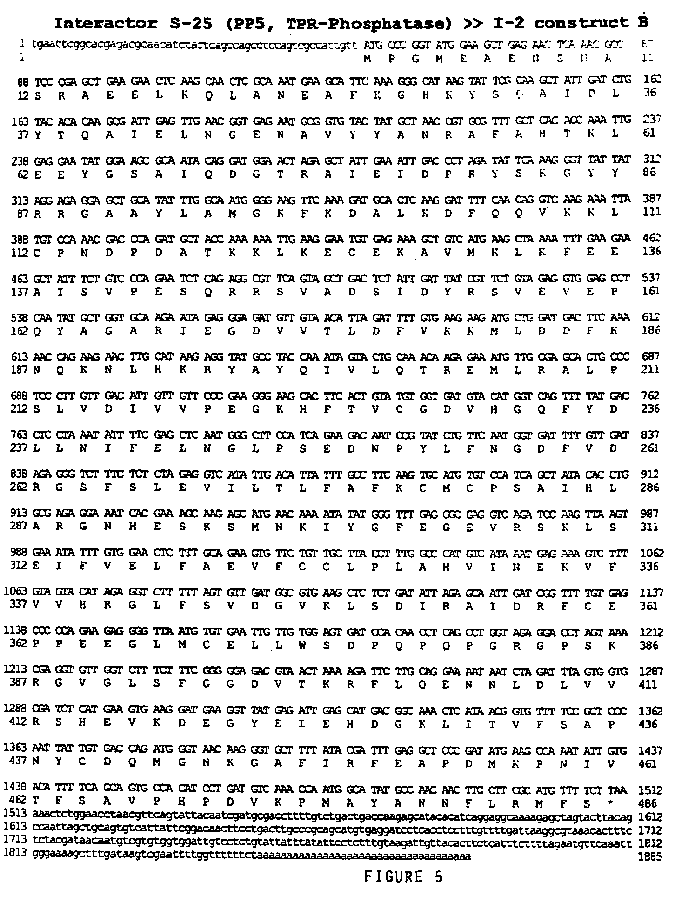

According to another embodiment, the invention relates to nucleotide sequences designated by interactor J-49 or interactor S-25 which are capable of interacting with the C-terminal domain of the I-2 polypeptide product. They are represented on figures 4 and 5.

The invention also concerns the above-defined nucleotide sequences which are modified and especially mutated, in order to alter their activity in the defence reaction against plant pathogens. According to another embodiment, the invention is also directed to nucleotide sequences which are modified and especially mutated in order to alter their specificity in the defence reaction against plant pathogens.

The invention is also directed to fragments of the described nucleotide sequences or to amino acid sequences contained within the described polypeptide sequences, provided they present the functional properties of the original sequence, of interest within the scope of the present invention. Advantageously said fragments bear a binding function involved in the interaction with polypeptide products produced by plants in the signal transduction pathway of resistance to pathogens.

Advantageously, such nucleotide fragments hybridize with the active sequence of the original sequence.

The invention also concerns nucleotide fragments or polypeptide fragments identified in a nucleotide sequence of the invention, in a determined plant, which have similarities with sequences obtained from other plants and especially which share common or conserved nucleotide strands or polypeptide strands with others.

To the contrary, the invention also encompasses nucleotide or polypeptide fragments which are specific of a determined plant species, or different with respect to other species when compared with sequences having similarities in other plant species.

Related or to the contrary specific domains in the compared sequences, may be identified upon using antibodies, especially monoclonal antibodies, directed against a determined domain, especially a functional domain of said sequence and detecting the presence or respectively absence of cross-reactions between the polypeptide products of various sequences.

According to the invention, the activity or the specificity of the nucleotide sequence in the defence reaction against plant pathogens can be altered either to improve said activity or specificity or to the contrary to lower said activity or specificity, especially where a broad spectrum of resistance in plants is required.

Examples of modification and especially of mutation are disclosed in the experimental data which are described hereafter. Such modification can for example affect the phosphatase activity of the interactor protein.

The invention also relates to a recombinant nucleotide sequence which comprises a nucleotide sequence according to one or several of the above definitions, said nucleotide sequence being under the control of a pathogen induced promoter.

The recombinant nucleotide sequence according to the invention is in particular a sequence wherein the promoter is a foliar or root pathogen promoter especially an inducible promoter.

As examples of pathogen induced promoters, the following may be used: CAMV35S promoter, potato GST-promoter 12 promoter.

The invention also concerns a recombinant vector comprising a nucleotide sequence according to one or several of the above definitions and it is especially directed to a recombinant vector wherein said nucleotide sequence is cloned in antisense orientation.

The invention further relates to cells transformed with a nucleotide sequence or a recombinant sequence or a vector of the invention, especially cells which are selected among bacteria, yeasts and plant cells.

The invention is also directed to plants transformed with a nucleotide sequence as defined above or with a recombinant sequence or vector replying to one or several of the above definitions.

Plants can be selected for transformation with the nucleotide sequence of the invention among those which are susceptible to the soil plant pathogen whose signaling gene in plants has been isolated.

The invention enables transformation of plants with said nucleotide sequence or transformation of plant parts including plant parts required for the multiplication or reproduction of the plant.

Polypeptide products are also within the frame of the invention, especially those defined as the expression product of a nucleotide sequence of the invention.

Such a polypeptide product of the invention is the product being active in signal transduction in the defence of plants against a plant pathogen, and being capable of interacting with a polypeptide encoded by said plant pathogen.

Advantageously, such a polypeptide product is capable of interacting with the N-terminal or with the C-terminal part of the polypeptide product encoded by the *I-2* resistance gene.

Among said polypeptide products interacting with the polypeptide product encoded by the *I-2* resistance gene, the invention encompasses those selected among Interactor K-10, Interactor K-23, Interactor K-6, Interactor J-49, Interactor S-25.

According to the present invention, the nucleotide sequence can be used for the activation of defence reactions against plant pathogens. It can especially be used for the activation according to the definition given above, wherein the nucleotide sequence described above, is cloned in antisense orientation.

Plant species that may be transformed in order to express a defence reaction against plant pathogens include those which are cited in the examples and further include plants selected among all higher plants, for example monocotyledon or dicotyledon plants, including solanaceae, compositae, cucurbitaceae, brassicaceae, gramineae.

According to a particular embodiment of the invention, R-gene-independent activation of defence is sought. To achieve this, two approaches are illustrated hereafter.
1) The nucleotide sequences of the invention (so-called signaling genes) are expressed using a pathogen-inducible promoter. Expression of the downstream signaling component of the signaling pathway occurs at the site of pathogen invasion with a different timing and at a higher level than normally. Depending on the promoter used the localized activation of defence responses can result in resistance against foliar or root pathogens. Examples of appropriate promoters are potato GST, 12, CAMV35S.
2) Signaling genes with a conserved protein structure are engineered to either a dominant negative or a constitutively active form. Mutations in conserved amino acid motifs that alter the biochemical properties of a protein have been described for many signaling components (kinases, phosphatases, G-proteins, transcription factors etc.).

The combination of altered expression patterns and biochemical properties of common signaling genes can be applied. Because no specific recognition is required, breaking-down of resistance is less likely to occur, resulting in durable resistance .

Figures 1 to 5 represent nucleotide sequences and corresponding aminoacid sequences of I-2 interactors.

Figures 1 to 3 represent interactor's nucleotide sequences which encode polypeptide products which recognize the N-terminal part of the I-2 polypeptide product.

Figures 4 and 5 represent interactor's nucleotide sequences which encode polypeptide products which recognize the C-terminal part of the I-2 polypeptide product.

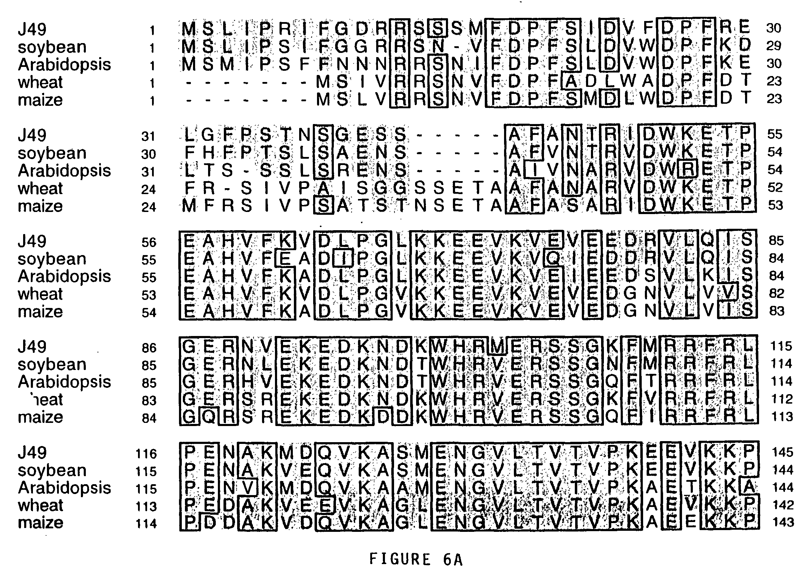

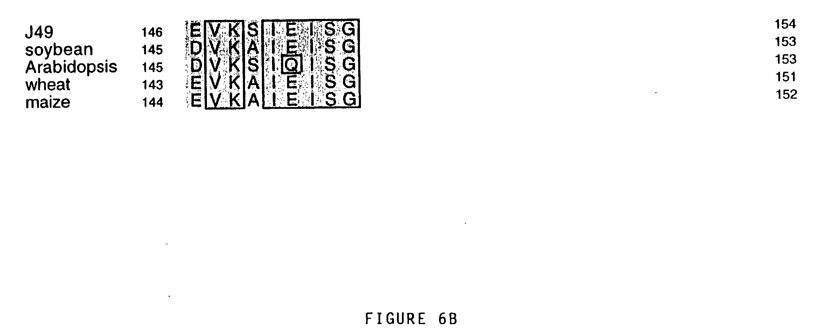

Figure 6. Alignment of the protein sequence from clone J49 with proteins from different plant origin. The Soybean sequence was derived from accession number P04793, the Arabidopsis sequence was derived from accession number AAC95188, the Wheat sequence was derived from accession number P12810, the Maize sequence was derived from accession number S23212.

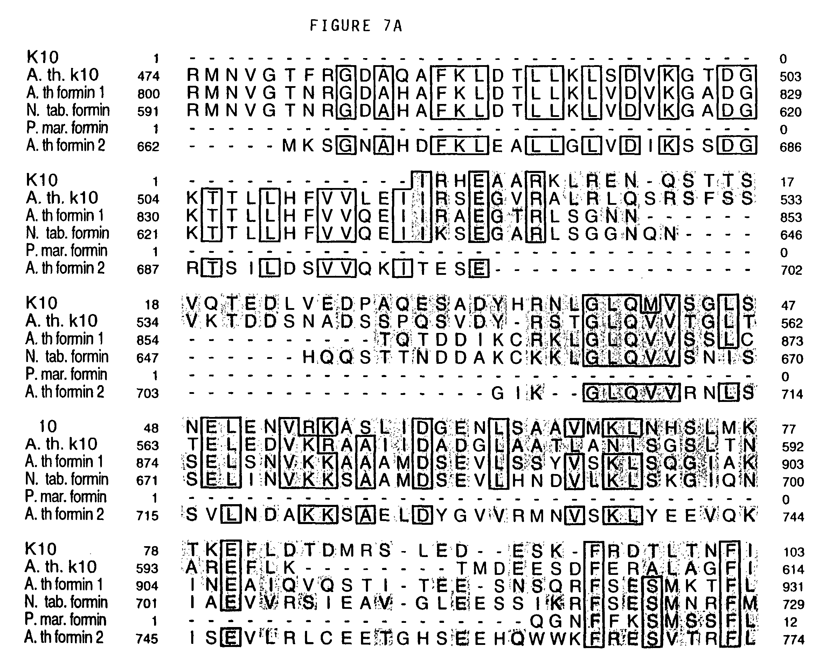

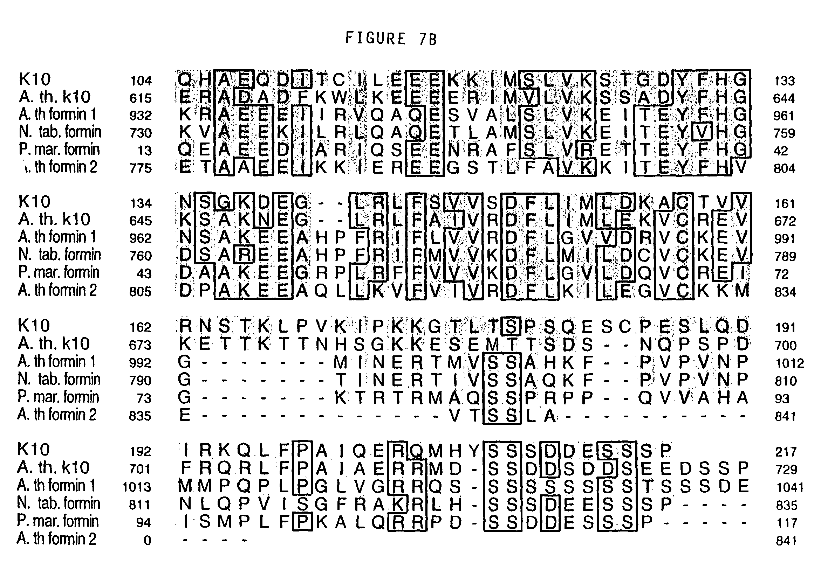

Figure 7. Alignment of the protein sequence from clone K10 with proteins from different plant origin. The Arabidopsis K10 sequence was derived from accession number Z97338, the Arabidopsis forming 1 sequence was derived from accession number AAF145548, the Nicotiana tabacum sequence was derived from accession number AAF24497, the Picea mariana sequence was derived from accession number AAC32145, the Arabidopsis forming 2 sequences was derived from accession number AAF02158.

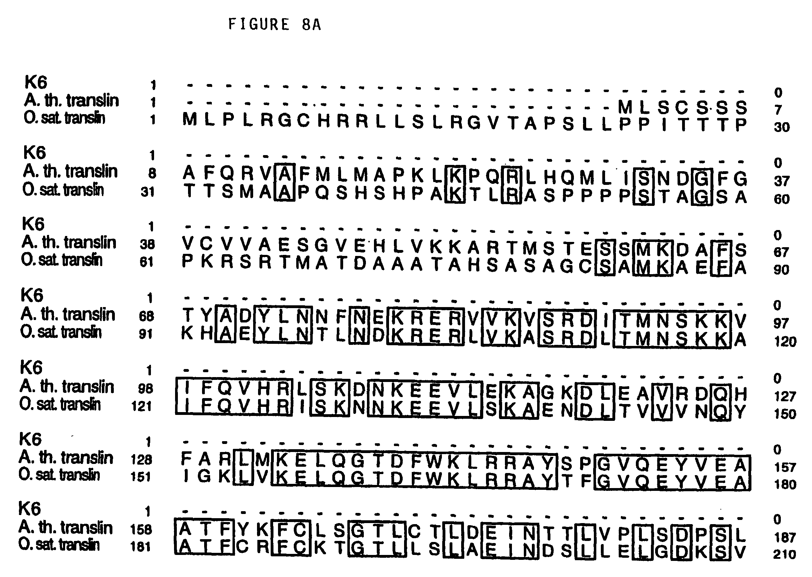

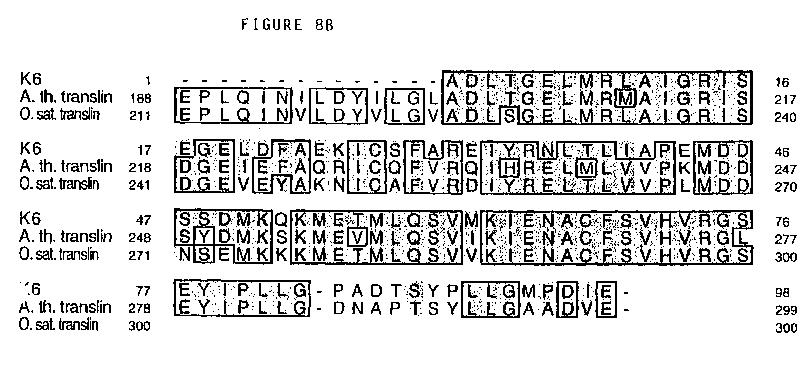

Figure 8. Alignment of the protein sequence from clone K6 with proteins from different plant origin. The Arabidopsis sequence was derived from accession number AAD20080 and the Oryza sativa sequence was derived from accession number BAA90355.

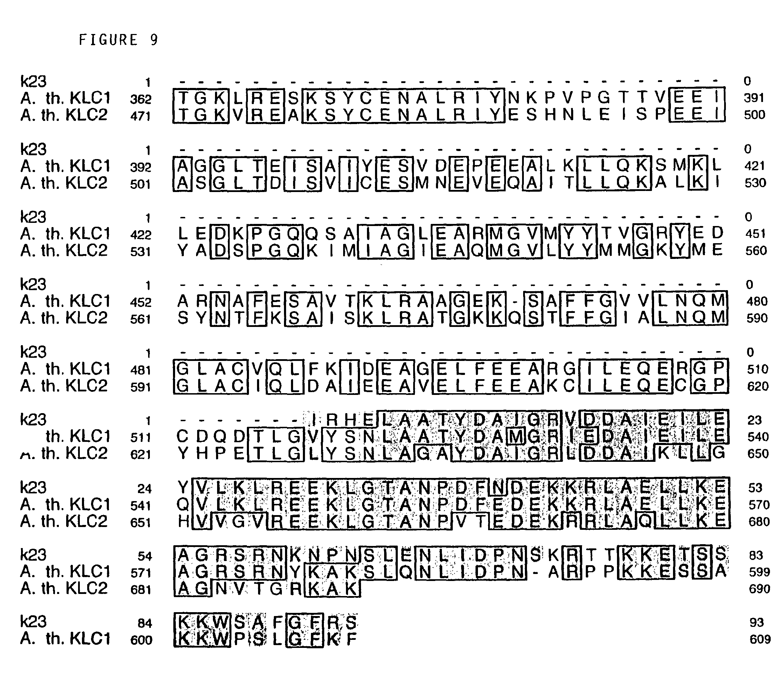

Figure 9. Alignment of the protein sequence from clone K23 with different plant proteins. The Arabidopsis KLC1 sequence was derived from accession number T01892 and the Arabidopsis KLC2 sequence was derived from accession number AAC80630.

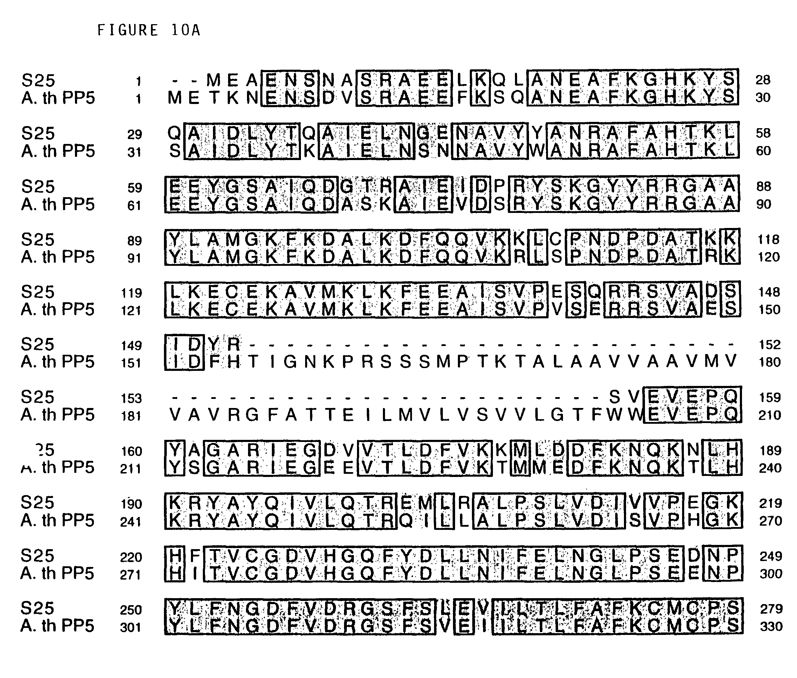

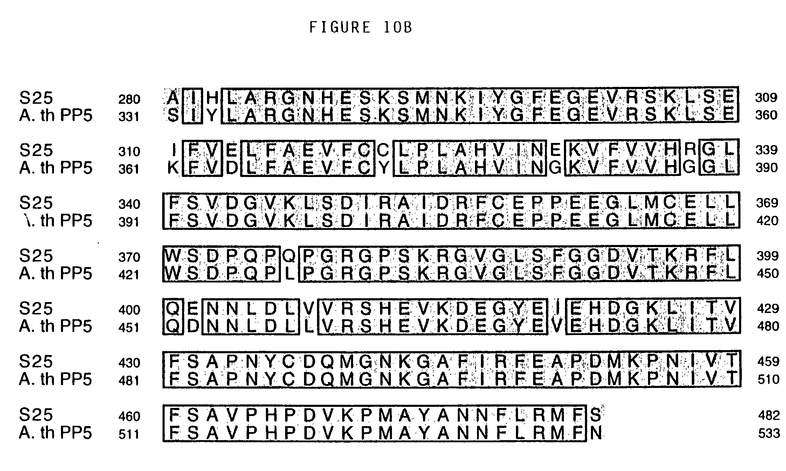

Figure 10. Alignment of the protein sequence from clone S25 with an Arabidopsis protein sequence that was derived from accession number AAD21727.

Other features and advantages of the invention can be derived from the following examples and drawings.

### Experimental part

### Introduction

As a model for race-specific recognition of pathogens by plants, the inventors have studied the interaction between tomato (*Lycopersicon esculentum*) and the causal agent of *Fusarium* wilt, the fungus *Fusarium oxysporum.* Tomato is the only host for the *forma specialis* (f.sp.) *lycopersici* of this worldwide ocurring, soil-borne pathogen. Three races of *F. oxysporum* f.sp. *lycopersici* (Fol) have been described. Recently the inventors have shown interactions between isolates of races of Fol and tomato plants carrying various resistance genes giving evidence for the existence of a so-called gene-for-gene relationship between them (Mes *et al.,* 1999). According to the gene-for-gene hypothesis (Flor, 1971; Keen, 1990), a pathogen-derived ligand (a race-specific elicitor encoded by an avirulence gene) is recognised by a plant receptor encoded by a correponding R-gene from the plant. Binding the elicitor activates the receptor or receptor complex. During this process, interactions of the receptor with other proteins are believed to occur or components of the receptor complex may be (de)phosphorylated. In this way an intracellular signal transduction cascade is triggered, leading to an efficient defence response (Staskawicz *et al.,* 1995; Song *et al*., 1995).

The primary structures of the products of cloned R-genes seem to confirm the receptor-like character of these proteins (Staskawicz *et al.,* 1995; Grant *et al.,* 1995). With one exception (pto), all R-gene products contain a leucine rich repeat region (LRR). Leucine rich repeats in general are involved in protein-protein interactions. Low conservation is observed in the primary sequence but the three dimensional structure is believed to be very similar. Such a variability enables the recognition of very diverse ligands, and therefor the LRR region is believed to be a receptor domain for pathogen derived ligands. Many R-gene products contain besides a C-terminal LRR a central nucleotide binding site (NBS) and a variable N-terminus. The N-terminus is believed to cause the specificity of downstream signaling. A set of R-gene products that contain a leucine zipper (LZ) motif in their N-termini seem to activate the same signaling cascade. A different signaling cascade is activated by a set of R-gene products with a so called TIR (Toll Interleukin receptor Related) domain in their N-termini (Aarts *et al.,* 1998). The N-terminus of *I-2* belongs to a new class that is characterised by a high percentage of charged amino acid residues.

### Methodology

The inventors have used the yeast two-hybrid system (Field and Song, 1989; Gyuris *et al.,* 1993) to clone genes encoding proteins that physically interact with the *I-2* gene product. In the following example, the two-hybrid system proposed by CLONTECH under the name MATCHMAKER GAL4 has been used, in accordance with the yeast Protocol Handbook and the MATCHMAKER Vectors Handbook of the manufacturer. The sequences encoding the 870 N-terminal amino acids (construct A) and the 750 C-terminal amino acids (construct B) of the *I-2* protein were fused to the *GAL4* binding domain (BD) in the yeast shuttle vector pAS2-1. The two fusion proteins were highly expressed in the yeast strain PJ69-4A, indicating that these strains were suitable for interaction cloning. A prey library was constructed in the λACT-II vector (Memelink J. et al, Elsevier Trends Journals Technical Tips Online, April 2000) using cDNA made from mRNA isolated from stem and root tissue of a susceptible tomato cultivar that had been inoculated with Fol race 2. A saturated screen of this library in both yeast strains harbouring the bait plasmids yielded five transformants that expressed all three marker genes (*HIS3. LacZ*, *ADE2*). This activation was confirmed to depend on the presence of the I-2 bait. Southern blotting revealed that all clones originated from tomato and not from the fungus. The inserts of the plasmids have been sequenced and all five clones showed homology to sequences in the database (see Figures 1 to 5).
- Interactors with the N-terminal domain of *I-2*:
   Interactor K-10 (diaphanous/formin homolog)
   Interactor K-23 (kinesin light chain homolog)
   Interactor K-6 (Translin homolog)
- Interactors with the C-terminal domain of *I-2*:
   Interactor J-49 (Hsp17)
   Interactor S-25 (PP5, TPR-Phosphatase homolog)
   Members of the pp5 are involved in receptor mediated signaling. Besides a C-terminal catalytical domain members of this family contain four N-terminal tetratricopeptide repeat (TPR) motifs. This domain has a regulatory function since binding of Arachidonic acid to this domain increases the phosphatase activity 25 fold in mammalian cells (Chen and Cohen 1997). In addition, there is evidence that TPR domains are involved in interactions with other proteins. In agreement with our hypothesis that different R-genes make use of the same signal transduction components, we showed that clone 525 could also bind to the *Arabidopsis RPM1* protein in the yeast two-hybrid system.

The fact that Arabidopsis homologs are present for all clones indicates that even distantly related plant species have similar signal transduction components, allowing their application to a wide range of crop plants

### Use of the isolated interactor genes

### - Anti-sense expression of interactors in resistant and susceptible tomato lines

One member of each group will be cloned in an antisense orientation behind the CaMV35S promoter in a binary vector. The constructs will be transformed into a tomato cultivar, which carries several resistance genes, next to *I* and *I-2* (also a susceptible cultivar will be selected and transformed with the antisense constructs). Thirty independent transgenic lines will be generated for each anti-sense construct. As a control for the effectiveness of the anti-sense strategy the *I-2* gene will be cloned into the same vector in anti-sense orientation (previous work has shown that this approach is feasible, Ori *et al.,* 1997). The F1-progeny of these lines will be tested for loss of resistance against Fol race 2. Lines that show suppression of the *I-2* based resistance will subsequently be tested for loss of resistance encoded by *I* and other resistance genes.

It should be noted that the anti-sense appoach mentioned above is effective only for genes that positively affect the defence response. The identification of negative regulators of defence responses can be achieved by screening transgenic susceptible plant lines with several pathogens, for example Fol race 1, 2 and 3 or *Cladosporium fulvum.* Gain of resistance would indicate the involvement of the interactor in defence responses as a negative regulator of the signaling cascade.

### - Pathogen inducible expression of positive regulators in a susceptible tomato line

When the functionality of the I-2 interactors in defence signaling has been assesed, full length cDNAs encoding interactors that act as positive regulators of defence responses will be fused to a pathogen inducible promoter in a binary vector. Negative regulators can be cloned in antisense orientation. The inventors propose to use the potato GST-promoter for this purpose as the activity of this promoter has been described in detail and the data suggest that it can give a high level of pathogen-induced expression (Strittmatter *et al.,* 1996). Altenatively, other promoters can be used for this purpose. These gene constructs will be introduced into a susceptible tomato line in order to achieve resistance against root and/or foliar pathogens

### - Construct modified signaling genes

The primary structure of the characterized interactors can provide alternative strategies to stimulate plant defence responses. Dominant alleles with altered activity or specificity will be constructed. For example, in case of the interacting phospatase (clone S-25), it will be attempted to generate alleles that cause increased phosphatase activity. This can be done either by deletion of the negative regulating TPR domain (Ramer and Davis, 1993; Maeda *et al.,* 1995), or by *in vitro* mutagenesis (Brunner *et al.,* 1994). Mutant alleles can be tested for their increased activity in an *E. coli* expression system. If other known signaling proteins will be cloned (kinases or G-proteins), site directed mutagenesis of specific amino-acids will yield proteins that are constitutively active (Bourne *et al.,* 1991). It has been shown previously that constitutive G-protein activation by cholera toxin can cause increased resistance in tobacco against *Pseudomonas* (Beffa *et al.* 1995). Besides constructing constitutive active alleles of positive activators, it is also possible to construct dominant negative alleles of negative regulators of the defence response (Chen *et al*., 1994).

Finally, it is clear that the interaction domain of the two-hybrid selected protein plays an important role in signal transduction and is a target for modification. Depending on the nature of the identified protein, this domain can be characterized for the individual proteins and *in vitro* mutagenesis can be carried out to select for mutations that enhance or abolish the interaction with the *I-2* protein.

### References

- Aarts N., Metz, M., Holub, E.B., Staskawicz B.J., Daniels, M.J. and Parker, J.E. (1998) Different requirements for *EDS1* and *NDR1* by disease resistance genes define at least two R gene-mediated signaling pathways in Arabidopsis. Proc. Natl. Acad. Sci USA 95: 10306-10311.
- Beffa, R., Szell, M., Meuwly, P., Pay, A., Vogeli-Lange, R., Metraux, J.P., Neuhaus, G., Meins Jr, F. and Nagy, F. (1995). Cholera toxin elevates pathogen resistance and induces pathogenesis-related gene expression in tobacco. EMBO J. 14: 5753-5761.
- Bourne, H.R., Sanders, D.A. and McCormick, F. (1991). The GTPase superfamily: conserved structure and molecular mechanism. Nature 349: 117-127.
- Brunner, D., Oellers, N., Szabad, J., Biggs 3rd, W.H., Zipursky, S.L. andHafen, E. (1994). A gain-of-function mutation in Drosophila MAP kinase activates multiple receptor tyrosine kinase signaling pathways. Cell 76: 875-888.
- Claret, F.X., Hibi, M., Dhut, S., Toda, T. and Karin, M. (1996). A new group of conserved coactivators that increase the specificity of AP-1 transcription factors. Nature 383: 453-457.
- Chen, S. Y., Huff, S.Y., Lai, C.C., Der, S. and Powers, C. J. (1994). Ras-15A protein shares highly similar dominant-negative biological properties with Ras-17N and forms a stable, guanine-nucleotide resistant complex with CDC25 exchange factor. Oncogene: 9, 2691-2698.
- Chen, M.X. and Cohen, P.T.W. (1997) Activation of protein phosphatase 5 by limited proteolysis or the binding of polyunsaturated fatty acids to the TPR domain. FEBS Lett 400, 136-140.
- Field, S. and Song, O. (1989). A novel genetic system to detect protein-protein interactions. Nature 340: 245-246.
- Flor, H.H. (1971). Current status of gene-for-gene concept. Annu. Rev. Phytopathol. 9: 275-296.
- Grant, M.R., Godiard, L., Straube, E., Ashield, T., Leward, J., Sattler, A., Innes, R.W. and Dangl, J.L. (1995). Structure of the Arabidopsis RPM1 gene enabling dual specificity disease resistance. Science 269: 843-846.
- Gyuris, J., Golemis, E., Chertkov, H. and Brent, R. (1993). Cdi1, a human G1 and S phase protein phophatase that associated with Cdk2. Cell 75: 791-803.
- Keen, N.T. (1990). Gene-for-gene complementarity in plant-pathogen interactions. Annu. Rev. Genet. 24: 447-463.
- Loh, Y.-T. and Martin, G.B. (1995). The Pto bacterial resistance gene and the Fen insecticide sensitivity gene encode functional protein kinase with serine/threonine specificity. Plant Physiol. 108: 1735-1739.
- Maeda T., Takekawa, M. and Saito, H. (1995). Activation of yeast PBS2 MAPKK by MAPKKKs or by binding of an SH3-containing osmosensor. Science 269: 554-558.
- Memelink J., Elsevier Trends Journals Technical Tips Online, April 2000.
- Mes, J.J., Weststeijn, E.A., Herlaar, F., Lambalk, J.J.M. Wijbrandi, J., Haring, M.A. and Cornelissen, B.J.C. (1999) Biological and molecular characterization of *Fusarium oxysporum* f.sp. *lycopersici* divides race 1 isolates into separate virulence groups. Phytopathology 89:156-160.
- Ori, N., Eshed, Y., I. Paran, G. Presting, D. Aviv, S. Tanksley, D. Zamir and R. Fluhr. (1997). The I2C family from the wilt disease resistance locus I2 belongs to the nucleotide binding, leucine-rich repeat superfamily of plant resistance genes. Plant Cell 9: 521-532.
- Ramer, S.W. and Davis, R.W. (1993). A dominant truncation allele identifies a gene, *STE20*, that encodes a putative protein kinase necessary for mating in *Saccharomyces cerevisiae.* Proc. Natl. Acad. Sci. USA 90: 452-456.
- Simons G., Groenendijk J., Wijbrandi J., Reijans M., Groenen J., Diergaarde P., Van der Lee T., Bleeker M., Onstenk J., De Both M., Haring M., Mes J., Cornelissen B., Zabeau M. and Vos P. (1998) Dissection of the *Fusarium I2* gene cluster reveals six homologs and one active gene copy. Plant Cell 10: 1055-1068.
- Strittmatter, G., Gheysen, G., Gianninazzi-Pearson, V., Hahn, K., Rohde, W. and Tacke, E (1996). Infections with various types of organisms stimulate transcription from a short promoter fragment of the potato GST1 gene. Mol. Plant Microbe Interact. 9: 68-73.
- Song, W.-Y., Wang, G.-L., Chen L.-L., Kim, H.-S., Pi, L.-Y., Holsten, T, Gardner, J., Wang, B., Zhai, W.-X., Zhu, L.-H., Fauquet, C. and Ronald, P. (1995). A receptor kinase-like protein encoded by the rice disease resistance gene, Xa21. Science 270: 1804-1806.
- Staskawicz, B.J., Ausubel, F.M., Baker, B.J., Ellis, J.G. and Jones, J.D.G. (1995). Molecular genetics of plant resistance. Science 268: 661-667.
- Zhou, J., Loh, Y.-T., Bressan, R.A.and Martin, G.B. (1995). The tomato gene Pti encodes a serine/threonine kinase that is phosphorylated by Pto and is involved in the Hypersensitive Response. Cell 83: 925-935.
- Zhou, J., Tang, X. and Martin, G.B. (1997). The Pto kinase conferring resistance to tomato bacterial speck disease interacts with proteins that bind a *cis*-element of pathogenesis-related genes. EMBO J. 16: 3207-3218.

## Claims

1. Nucleotide sequence derived from a plant genome, encoding a polypeptide product which can be activated as a signaling molecule in the signal transduction pathway of resistance to a plant pathogen which resistance results from the interaction between polypeptide products encoded by a resistance gene and elicitors encoded by an avirulence gene in said soil plant pathogen, wherein the polypeptide product encoded by the nucleotide sequence interacts with a polypeptide product encoded by said resistance gene.

2. Nucleotide sequence according to claim 1 derived from a plant genome, encoding a polypeptide product which can be activated as a signaling molecule in the signal transduction pathway of resistance to a plant pathogen, said plant pathogen being a soil plant pathogen.

3. Nucleotide sequence according to claim 1 or 2, encoding a polypeptide product that interacts with the N-terminal part or with the C-terminal part of the polypeptide product encoded by the resistance gene.

4. Nucleotide sequence derived from a plant genome, which encodes a polypeptide product that interacts with the polypeptide product encoded by the 1-2 resistance gene.

5. Nucleotide sequence according to anyone of claims 1 to 4, which encodes a polypeptide product that interacts with the N-terminal part of the polypeptide product encoded by the I-2 resistance gene.

6. Nucleotide sequence according to anyone of claims 1 to 4, which encodes a polypeptide product that interacts with the C-terminal part of the polypeptide product encoded by the I-2 resistance gene.

7. Nucleotide sequence according to anyone of claims 1 to 6, which is a cDNA sequence.

8. Nucleotide sequence according to anyone of claims 1 to 6, which encodes a signal transduction component.

9. Nucleotide sequence according to anyone of claims 1 to 7, which is the sequence of an Open Reading Frame.

10. Nucleotide sequence according to anyone of claims 1 to 9, which can be obtained by the process comprising:
a) cloning into a yeast strain, one or several vectors comprising a construct comprising at least one determined region of a resistance gene to a plant pathogen, encoding a polypeptide product of said resistance gene to a plant pathogen wherein said at least determined region of said resistance gene is fused to a sequence coding for a binding domain of a transcription factor capable of activating a reporter gene present in the vector and placed under the control of regulation sequences whose activation is dependent upon interaction of the binding domain and of the activation domain of the transcription factor,
b) cloning into one or several vectors, a nucleotide sequence or a cDNA library obtained from a plant, which may be susceptible to said plant pathogen or is naturally resistant to said plant pathogen or has been rendered resistant to said plant pathogen, the cloned nucleotide sequences being fused in the vector, with a nucleotide sequence encoding the activation domain of said transcription factor,
c) expressing said vectors of steps a) and b) in said yeast strain,
d) identifying positive clones resulting in activation of the marker gene cloned into the first or second vectors.

11. Nucleotide sequence according to claim 10, wherein the resistance gene is the I-2 gene.

12. Nucleotide sequence according to anyone of claims 1 to 11, which is selected among the sequence of Figure 1 designated Interactor K-10, the sequence of Figure 2 designated Interactor K-23, the sequence of Figure 3 designated Interactor K-6, the sequence of Figure 4 designated Interactor J-49, the sequence of Figure 5 designated Interactor S-25.

13. Nucleotide sequence according to anyone of claims 1 to 12, which is mutated to alter its activity in the defence reaction against plant pathogens.

14. Nucleotide sequence according to anyone of claims 1 to 12, which is mutated to alter its specificity in the defence reaction against plant pathogens.

15. Recombinant nucleotide sequence which comprises a nucleotide sequence according to anyone of claims 1 to 12 under the control of a promoter selected among a pathogen-induced promoter, promoters inducible by foliar or root pathogens, a pathogen inducible promoter.

16. Recombinant vector comprising a nucleotide sequence according to anyone of claims 1 to 15.

17. Recombinant vector according to claim 16, wherein the nucleotide sequence according to anyone of claims 1 to 12, is cloned in antisense orientation.

18. Cell transformed with a nucleotide sequence according to anyone of claims 1 to 17.

19. Cell according to claim 18 which is selected among bacteria, yeasts and plant cells.

20. Plant transformed with a nucleotide sequence according to anyone of claims 1 to 17.

21. Polypeptide product which is the expression product of a nucleotide sequence according to anyone of claims 1 to 17.

22. Polypeptide product being active in signal transduction in the defence mechanism of plants against a plant pathogen, and being capable of interacting with a polypeptide encoded by said plant pathogen.

23. Polypeptide product which is capable of interacting with the N-terminal or with the C-terminal part of the polypeptide product encoded by the I-2 resistance gene.

24. Polypeptide product according to anyone of claims 21 to 23 which is selected among Interactor K-10, Interactor K-23, Interactor K-6, Interactor J-49, Interactor S-25 polypeptide products.

25. Use of a nucleotide sequence according to anyone of claims 1 to 15 or of a polypeptide according to anyone of claims 21 to 24, for the activation of defence reactions against plant pathogens.

26. Use of a nucleotide sequence or of a polypeptide product according to anyone of claims 21 to 24, for the activation of defence reactions against plant pathogens.

27. Use of a nucleotide sequence according to claim 25 or of a polypeptide according to anyone of claims 23 to 26, for the activation of defense reactions against plant pathogens, independently of the activation of race-specific resistance genes.

28. Use according to claim 25 or 27 or of a polypeptide according to anyone of claims 21 to 24 in plant species selected among dicotyledon or monocotyledon plants.


### Annex to the application documents - subsequently filed sequences listing

SEQUENCE LISTING

<110> KEYGENE N.V.

<120> NUCLEOTIDE SEQUENCES CODING SIGNAL TRANSDUCTION
      COMPONENTS IN DURABLE AND BROAD-RANGE RESISTANCE
      STRATEGIES BASED ON PLANT DEFENCE.

<130> B4538_ AD

<140> 00401402.3
<141> 2000-05-19

<160> 28

<170> PatentIn Ver. 2.1

<210> 1
<211> 1255
<212> DNA
<213> Lycopersicon esculentum

<220>
<221> CDS
<222> Complement((1)..(777))

<220>
<221> misc_feature
<222> Complement((1)..(1255))
<223> Interactor K-10

<400> 1
tat cta ttc gat gat gaa gat acc cca cca aac cca aaa aaa gag atc    48
Tyr Leu Phe Asp Asp Glu Asp Thr Pro Pro Asn Pro Lys Lys Glu Ile
  1               5                  10                  15

tct atg gct tac cca tac gat gtt cca gat tac gct agc ttg ggt ggt    96
Ser Met Ala Tyr Pro Tyr Asp Val Pro Asp Tyr Ala Ser Leu Gly Gly
             20                  25                  30

cat atg gcc atg gag gcc ccg ggg atc cga att cgg cac gag gca gca    144
His Met Ala Met Glu Ala Pro Gly Ile Arg Ile Arg His Glu Ala Ala
         35                  40                  45

cgt aaa tta aga gag aac caa agc act acc agt gta cag aca gaa gat    192
Arg Lys Leu Arg Glu Asn Gln Ser Thr Thr Ser Val Gln Thr Glu Asp
     50                  55                  60

ctt gta gaa gat cct gct cag gag tcg gca gat tat cat cgt aac ctt 240
Leu Val Glu Asp Pro Ala Gln Glu Ser Ala Asp Tyr His Arg Asn Leu
65                  70                  75                  80

ggt ctt cag atg gtt tct ggt tta agc aac gag ctt gag aat gta aga 288
Gly Leu Gln Met Val Ser Gly Leu Ser Asn Glu Leu Glu Asn Val Arg
                85                  90                  95

aaa gct tca ctt att gac ggc gag aac tta agt gca gct gtc atg aag 336
Lys Ala Ser Leu Ile Asp Gly Glu Asn Leu Ser Ala Ala Val Met Lys
            100                 105                 110

ctt aat cac tca ctc atg aaa act aaa gag ttt ctg gac act gat atg 384
Leu Asn His Ser Leu Met Lys Thr Lys Glu Phe Leu Asp Thr Asp Met
        115                 120                 125

aga agt ttg gag gat gaa agt aag ttc cgt gat aca ctc aca aat ttt 432
Arg Ser Leu Glu Asp Glu Ser Lys Phe Arg Asp Thr Leu Thr Asn Phe
    130                 135                 140

atc caa cat gcg gaa cag gac att act tgc ata cta gaa gaa gag aaa 480
Ile Gln His Ala Glu Gln Asp Ile Thr Cys Ile Leu Glu Glu Glu Lys
145                 150                 155                 160

aag ata atg tct ttg gtt aag agc aca gga gat tac ttc cat gga aat 528
Lys Ile Met Ser Leu Val Lys Ser Thr Gly Asp Tyr Phe His Gly Asn
                165                 170                 175

tca ggg aag gat gaa ggc ttg cgt ctc ttt tca gtc gtt agt gat ttc 576
Ser Gly Lys Asp Glu Gly Leu Arg Leu Phe Ser Val Val Ser Asp Phe
            180                 185                 190

ttg att atg ttg gac aag gca tgt aca gtg gtg aga aac tca acg aag 624
Leu Ile Met Leu Asp Lys Ala Cys Thr Val Val Arg Asn Ser Thr Lys
        195                 200                 205

tta cca gtt aag att cct aaa aaa ggg aca tta aca tct cct tcc caa 672
Leu Pro Val Lys Ile Pro Lys Lys Gly Thr Leu Thr Ser Pro Ser Gln
    210                 215                 220

gaa tcc tgt cct gag tct ttg caa gac ata cgt aaa caa cta ttt cct 720
Glu Ser Cys Pro Glu Ser Leu Gln Asp Ile Arg Lys Gln Leu Phe Pro
225                 230                 235                 240

gca atc cag gag cga cag atg cac tat tct agt tca gac gac gag agc 768
Ala Ile Gln Glu Arg Gln Met His Tyr Ser Ser Ser Asp Asp Glu Ser
                245                 250                 255

tca agc ccg tagctgtcta tttaggtgtc agagtacttt ggagctaata 817
Ser Ser Pro

tggcttggat aatagtccac agaagtatgt atttaacgac tatatgaggc ctaaggtgtg 877

cctcctacac ccagttagca aacacatgct ctacgagata gagtgaatag acattttgct 937

tcaatgatct cttcgagtcc tagcactagc ggtaggaaga atggctatag tatagatgcc 997

atagtagtta ttgcatggtc tactacaacg atccacagag ttaaaccaca gcctctcact 1057

gtctgtagtc acctttatct ataacaatcc aaatatcgag atggtaatgg tactgaaatc 1117

tcctaaatat ttgttaaatt atatttagta tgtattggaa gttagaccag attcgtatag 1177

tttcagcttc tttttctcgt caaaaaaaaa aaaaaaaaaa ctcgagagat ctatgaatcg 1237

tagatactga aaaacccc 1255

<210> 2
<211> 259
<212> PRT
<213> Lycopersicon esculentum

<400> 2
Tyr Leu Phe Asp Asp Glu Asp Thr Pro Pro Asn Pro Lys Lys Glu Ile
1               5                   10                  15

Ser Met Ala Tyr Pro Tyr Asp Val Pro Asp Tyr Ala Ser Leu Gly Gly
            20                  25                  30

His Met Ala Met Glu Ala Pro Gly Ile Arg Ile Arg His Glu Ala Ala
        35                  40                  45

Arg Lys Leu Arg Glu Asn Gln Ser Thr Thr Ser Val Gln Thr Glu Asp
    50                  55                  60

Leu Val Glu Asp Pro Ala Gln Glu Ser Ala Asp Tyr His Arg Asn Leu
65                  70                  75                  80

Gly Leu Gln Met Val Ser Gly Leu Ser Asn Glu Leu Glu Asn Val Arg
                85                  90                  95

Lys Ala Ser Leu Ile Asp Gly Glu Asn Leu Ser Ala Ala Val Met Lys
            100                 105                 110

Leu Asn His Ser Leu Met Lys Thr Lys Glu Phe Leu Asp Thr Asp Met

115                 120                 125

Arg Ser Leu Glu Asp Glu Ser Lys Phe Arg Asp Thr Leu Thr Asn Phe
    130                 135                 140

Ile Gln His Ala Glu Gln Asp Ile Thr Cys Ile Leu Glu Glu Glu Lys
145                 150                 155                 160

Lys Ile Met Ser Leu Val Lys Ser Thr Gly Asp Tyr Phe His Gly Asn
                165                 170                 175

Ser Gly Lys Asp Glu Gly Leu Arg Leu Phe Ser Val Val Ser Asp Phe
            180                 185                 190

Leu Ile Met Leu Asp Lys Ala Cys Thr Val Val Arg Asn Ser Thr Lys
        195                 200                 205

Leu Pro Val Lys Ile Pro Lys Lys Gly Thr Leu Thr Ser Pro Ser Gln
    210                 215                 220

Glu Ser Cys Pro Glu Ser Leu Gln Asp Ile Arg Lys Gln Leu Phe Pro
225                 230                 235                 240

Ala Ile Gln Glu Arg Gln Met His Tyr Ser Ser Ser Asp Asp Glu Ser
                245                 250                 255

Ser Ser Pro

<210> 3
<211> 502
<212> DNA
<213> Lycopersicon esculentum

<220>
<221> CDS
<222> Complement((1)..(267))

<220>
<221> misc_feature
<222> Complement((1)..(502))
<223> Interactor K-23 (TPR-kinesin)

<400> 3
ctt gcc gcc act tat gat gct ata gga aga gtg gat gat gct att gag    48
Leu Ala Ala Thr Tyr Asp Ala Ile Gly Arg Val Asp Asp Ala Ile Glu

1               5                   10                  15

att ctg gag tac gtt ctt aaa ctg aga gaa gaa aaa ctc gga act gca    96
Ile Leu Glu Tyr Val Leu Lys Leu Arg Glu Glu Lys Leu Gly Thr Ala
            20                  25                  30

aat cct gat ttc aac gat gag aaa aag agg ctg gct gaa tta ttg aaa    144
Asn Pro Asp Phe Asn Asp Glu Lys Lys Arg Leu Ala Glu Leu Leu Lys
        35                  40                  45

gaa gca ggt aga tct cgg aac aaa aac ccg aat tcc tta gaa aat ctt    192
Glu Ala Gly Arg Ser Arg Asn Lys Asn Pro Asn Ser Leu Glu Asn Leu
    50                  55                  60

att gat cca aac tct aaa agg acg acg aag aaa gag act tca tca aag    240
Ile Asp Pro Asn Ser Lys Arg Thr Thr Lys Lys Glu Thr Ser Ser Lys
65                  70                  75                  80

aag tgg tct gca ttt ggc ttc aga agt tgattctttc tagcaagaag          287
Lys Trp Ser Ala Phe Gly Phe Arg Ser
                85

tttttctata acattgtaaa ggcacgaatg tgtgttatgt tttatgtcac tataaactgg 347

tgaagcttat tggttgttgt ccttttgaat tcatgtcatt tatctttttt ttcattcatt 407

gcttgattgt ttatcagttt gtaatttgat gatgtttctg tttcaacaaa accaatcaat 467

gtttatggaa gtaaaaaaaa aaaaaaaaaa aaaaa                            502

<210> 4
<211> 89
<212> PRT
<213> Lycopersicon esculentum

<400> 4
Leu Ala Ala Thr Tyr Asp Ala Ile Gly Arg Val Asp Asp Ala Ile Glu
1               5                   10                  15

Ile Leu Glu Tyr Val Leu Lys Leu Arg Glu Glu Lys Leu Gly Thr Ala
            20                  25                  30

Asn Pro Asp Phe Asn Asp Glu Lys Lys Arg Leu Ala Glu Leu Leu Lys
        35                  40                  45

Glu Ala Gly Arg Ser Arg Asn Lys Asn Pro Asn Ser Leu Glu Asn Leu
    50                  55                  60

Ile Asp Pro Asn Ser Lys Arg Thr Thr Lys Lys Glu Thr Ser Ser Lys
65                  70                  75                  80

Lys Trp Ser Ala Phe Gly Phe Arg Ser
                85

<210> 5
<211> 744
<212> DNA
<213> Lycopersicon esculentum

<220>
<221> CDS
<222> Complement((1)..(306))

<220>
<222> ()..)

<220>
<221> misc_feature
<222> Complement((1)..(744))
<223> Interactor K-6 (Translin)

<400> 5
att cgg cac gag gcg gac ttg aca gga gaa tta atg agg tta gca atc    48
Ile Arg His Glu Ala Asp Leu Thr Gly Glu Leu Met Arg Leu Ala Ile
1               5                   10                  15

ggt cga att tca gaa ggg gaa ctt gat ttt gca gag aag atc tgc agt    96
Gly Arg Ile Ser Glu Gly Glu Leu Asp Phe Ala Glu Lys Ile Cys Ser
            20                  25                  30

ttt gcg cgt gaa att tac agg aac ctt act ctt att gcc cca gag atg    144
Phe Ala Arg Glu Ile Tyr Arg Asn Leu Thr Leu Ile Ala Pro Glu Met
        35                  40                  45

gat gat agt tca gac atg aaa cag aaa atg gaa aca atg ctc cag agt    192
Asp Asp Ser Ser Asp Met Lys Gln Lys Met Glu Thr Met Leu Gln Ser
    50                  55                  60

gtg atg aag ata gaa aat gct tgt ttt agt gtt cat gta aga gga tcg    240
Val Met Lys Ile Glu Asn Ala Cys Phe Ser Val His Val Arg Gly Ser
65                  70                  75                  80

gag tat att ccc ctt ctt gga cct gct gat acc agt tat cca ctg ttg    288

Glu Tyr Ile Pro Leu Leu Gly Pro Ala Asp Thr Ser Tyr Pro Leu Leu
                85                  90                  95

ggc atg cca gac att gaa tgaagaagca cgaaagataa gttccttgtt 336
Gly Met Pro Asp Ile Glu
            100

tgctgttgaa gttgttgatg cagctctccc agcctgcata tacacacggg gcagagcgtc 396

tttgttgggc tgcacttccg catgtgcttt tgcaaggtac tttggtgcaa cgatttgcat 456

cttcgtattt gaaattgaca cattttgtaa ttactccagt ccacctagtg aactttatgc 516

gactttgtag cggagattgt ataggcttac aagtactcta tttccctcct actgacctac 576

aagatggata ccttttttgc gaaatcgttg ccagagtttg ttttctgaga tgattttaga 636

ctagtacaca ctctttattt acccagaagg tattctgaat caatgttctt tttgttgtta 696

ttattgtatt tcatagcttt gaaacttaaa aaaaaaaaaa aaaaaaaa 744

<210> 6
<211> 102
<212> PRT
<213> Lycopersicon esculentum

<400> 6
Ile Arg His Glu Ala Asp Leu Thr Gly Glu Leu Met Arg Leu Ala Ile
1               5                   10                  15

Gly Arg Ile Ser Glu Gly Glu Leu Asp Phe Ala Glu Lys Ile Cys Ser
            20                  25                  30

Phe Ala Arg Glu Ile Tyr Arg Asn Leu Thr Leu Ile Ala Pro Glu Met
        35                  40                  45

Asp Asp Ser Ser Asp Met Lys Gln Lys Met Glu Thr Met Leu Gln Ser
    50                  55                  60

Val Met Lys Ile Glu Asn Ala Cys Phe Ser Val His Val Arg Gly Ser
65                  70                  75                  80

Glu Tyr Ile Pro Leu Leu Gly Pro Ala Asp Thr Ser Tyr Pro Leu Leu
                85                  90                  95

Gly Met Pro Asp Ile Glu
            100

<210> 7
<211> 296
<212> DNA
<213> Lycopersicon esculentum

<220>
<221> CDS
<222> Complement((52)..(294))

<220>
<221> misc_feature
<222> Complement((1)..(296))
<223> Interactor J-49 (Hsp17)

<400> 7
cgaattcggc acgagaaaaa acgtagaaaa ttctcaaaaa gttcactgaa a atg tct 57
Met Ser
1

ctg atc cca aga att ttc ggc gat cga cga agc agc agc atg ttc gat 105
Leu Ile Pro Arg Ile Phe Gly Asp Arg Arg Ser Ser Ser Met Phe Asp
        5                   10                  15

cca ttt tca att gac gta ttt gat cca ttc agg gaa tta ggc ttc cca 153
Pro Phe Ser Ile Asp Val Phe Asp Pro Phe Arg Glu Leu Gly Phe Pro
    20                  25                  30

agt acc aat tca ggg gag agc tct gca ttt gcc aac aca cga ata gac 201
Ser Thr Asn Ser Gly Glu Ser Ser Ala Phe Ala Asn Thr Arg Ile Asp
35                  40                  45                  50

tgg aag gaa act cca gaa gct cat gtg ttc aag gtt gat ctt cca ggg 249
Trp Lys Glu Thr Pro Glu Ala His Val Phe Lys Val Asp Leu Pro Gly
                55                  60                  65

ctt aag aag gag gaa gtc aaa gtg gaa gtc gag gag gaa ata ggg gt 296
Leu Lys Lys Glu Glu Val Lys Val Glu Val Glu Glu Glu Ile Gly
            70                  75                  80

<210> 8
<211> 81
<212> PRT
<213> Lycopersicon esculentum

<400> 8
Met Ser Leu Ile Pro Arg Ile Phe Gly Asp Arg Arg Ser Ser Ser Met
1               5                   10                  15

Phe Asp Pro Phe Ser Ile Asp Val Phe Asp Pro Phe Arg Glu Leu Gly
            20                  25                  30

Phe Pro Ser Thr Asn Ser Gly Glu Ser Ser Ala Phe Ala Asn Thr Arg
        35                  40                  45

Ile Asp Trp Lys Glu Thr Pro Glu Ala His Val Phe Lys Val Asp Leu
    50                  55                  60

Pro Gly Leu Lys Lys Glu Glu Val Lys Val Glu Val Glu Glu Glu Ile
65                  70                  75                  80

Gly

<210> 9
<211> 1885
<212> DNA
<213> Lycopersicon esculentum

<220>
<221> CDS
<222> Complement((55)..(1509))

<220>
<221> misc_feature
<222> Complement((1)..(1885))
<223> Interactor S-25 (PP5, TPR-Phosphatase)

<400> 9
tgaattcggc acgagacgca acatctactc agccagcctc cagtcgccat tgtt atg    57
                                                            Met
                                                            1

ccc ggt atg gaa gct gag aac tca aac gcc tcc cga gct gaa gaa ctc    105
Pro Gly Met Glu Ala Glu Asn Ser Asn Ala Ser Arg Ala Glu Glu Leu
            5                   10                  15

aag caa ctc gca aat gaa gca ttc aaa ggg cat aag tat tcg caa gct    153
Lys Gln Leu Ala Asn Glu Ala Phe Lys Gly His Lys Tyr Ser Gln Ala
        20                  25                  30

att gat ctg tac aca caa gcg att gag ttg aac ggt gag aat gcg gtg 201
Ile Asp Leu Tyr Thr Gln Ala Ile Glu Leu Asn Gly Glu Asn Ala Val
    35                  40                  45

tac tat gct aac cgt gcg ttt gct cac acc aaa ttg gag gaa tat gga 249
Tyr Tyr Ala Asn Arg Ala Phe Ala His Thr Lys Leu Glu Glu Tyr Gly
50                  55                  60                  65

agc gca ata cag gat gga act aga gct att gaa att gac cct aga tat 297
Ser Ala Ile Gln Asp Gly Thr Arg Ala Ile Glu Ile Asp Pro Arg Tyr
                70                  75                  80

tca aag ggt tat tat agg aga gga gct gca tat ttg gca atg ggg aag 345
Ser Lys Gly Tyr Tyr Arg Arg Gly Ala Ala Tyr Leu Ala Met Gly Lys
            85                  90                  95

ttc aaa gat gca ctc aag gat ttt caa cag gtc aag aaa tta tgt cca 393
Phe Lys Asp Ala Leu Lys Asp Phe Gln Gln Val Lys Lys Leu Cys Pro
        100                 105                 110

aac gac cca gat gct acc aaa aaa ttg aag gaa tgt gag aaa gct gtc 441
Asn Asp Pro Asp Ala Thr Lys Lys Leu Lys Glu Cys Glu Lys Ala Val
    115                 120                 125

atg aag cta aaa ttt gaa gaa gct att tct gtc cca gaa tct cag agg 489
Met Lys Leu Lys Phe Glu Glu Ala Ile Ser Val Pro Glu Ser Gln Arg
130                 135                 140                 145

cgt tca gta gct gac tct att gat tat cgt tct gta gag gtg gag cct 537
Arg Ser Val Ala Asp Ser Ile Asp Tyr Arg Ser Val Glu Val Glu Pro
                150                 155                 160

caa tat gct ggt gca aga ata gag gga gat gtt gta aca tta gat ttt 585
Gln Tyr Ala Gly Ala Arg Ile Glu Gly Asp Val Val Thr Leu Asp Phe
            165                 170                 175

gtg aag aag atg ctg gat gac ttc aaa aac cag aag aac ttg cat aag 633
Val Lys Lys Met Leu Asp Asp Phe Lys Asn Gln Lys Asn Leu His Lys
        180                 185                 190

agg tat gcc tac caa ata gta ctg caa aca aga gaa atg ttg cga gca 681
Arg Tyr Ala Tyr Gln Ile Val Leu Gln Thr Arg Glu Met Leu Arg Ala
    195                 200                 205

ctg ccc tcc ctt gtt gac att gtt gtt ccc gaa ggg aag cac ttc act 729
Leu Pro Ser Leu Val Asp Ile Val Val Pro Glu Gly Lys His Phe Thr
210                 215                 220                 225

gta tgt ggt gat gta cat ggt cag ttt tat gac ctc cta aat att ttc 777
Val Cys Gly Asp Val His Gly Gln Phe Tyr Asp Leu Leu Asn Ile Phe
                230                 235                 240

gag ctc aat ggg ctt cca tca gaa gac aat ccg tat ctg ttc aat ggt 825
Glu Leu Asn Gly Leu Pro Ser Glu Asp Asn Pro Tyr Leu Phe Asn Gly
            245                 250                 255

gat ttt gtt gat aga ggg tct ttc tct cta gag gtc ata ttg aca tta 873
Asp Phe Val Asp Arg Gly Ser Phe Ser Leu Glu Val Ile Leu Thr Leu
        260                 265                 270

ttt gcc ttc aag tgc atg tgt cca tca gct ata cac ctg gcg aga gga 921
Phe Ala Phe Lys Cys Met Cys Pro Ser Ala Ile His Leu Ala Arg Gly
    275                 280                 285

aat cac gaa agc aag agc atg aac aaa ata tat ggg ttt gag ggc gag 969
Asn His Glu Ser Lys Ser Met Asn Lys Ile Tyr Gly Phe Glu Gly Glu
290                 295                 300                 305

gtc aga tcc aag tta agt gaa ata ttt gtg gaa ctc ttt gca gaa gtg 1017
Val Arg Ser Lys Leu Ser Glu Ile Phe Val Glu Leu Phe Ala Glu Val
                310                 315                 320

ttc tgt tgc tta cct ttg gcc cat gtc ata aat gag aaa gtc ttt gta 1065
Phe Cys Cys Leu Pro Leu Ala His Val Ile Asn Glu Lys Val Phe Val
            325                 330                 335

gta cat aga ggt ctt ttt agt gtt gat ggc gtg aag ctc tct gat att 1113
Val His Arg Gly Leu Phe Ser Val Asp Gly Val Lys Leu Ser Asp Ile
        340                 345                 350

aga gca att gat cgg ttt tgt gag ccc cca gaa gag ggg tta atg tgt 1161
Arg Ala Ile Asp Arg Phe Cys Glu Pro Pro Glu Glu Gly Leu Met Cys
    355                 360                 365

gaa ttg ttg tgg agt gat cca caa cct cag cct ggt aga gga cct agt 1209
Glu Leu Leu Trp Ser Asp Pro Gln Pro Gln Pro Gly Arg Gly Pro Ser
370                 375                 380                 385

aaa cga ggt gtt ggt ctt tct ttc ggg gga gac gta act aaa aga ttc 1257
Lys Arg Gly Val Gly Leu Ser Phe Gly Gly Asp Val Thr Lys Arg Phe
                390                 395                 400

ttg cag gaa aat aat cta gat tta gtg gtg cga tct cat gaa gtg aag 1305
Leu Gln Glu Asn Asn Leu Asp Leu Val Val Arg Ser His Glu Val Lys
            405                 410                 415

gat gaa ggt tat gag att gag cat gac ggc aaa ctc ata acg gtg ttt 1353
Asp Glu Gly Tyr Glu Ile Glu His Asp Gly Lys Leu Ile Thr Val Phe
        420                 425                 430

tcc gct ccc aat tat tgt gac cag atg ggt aac aag ggt gct ttt ata 1401
Ser Ala Pro Asn Tyr Cys Asp Gln Met Gly Asn Lys Gly Ala Phe Ile
    435                 440                 445

cga ttt gag gct ccc gat atg aag cca aat att gtg aca ttt tca gca 1449
Arg Phe Glu Ala Pro Asp Met Lys Pro Asn Ile Val Thr Phe Ser Ala
450                 455                 460                 465

gtg cca cat cct gat gtc aaa cca atg gca tat gcc aac aac ttc ctt 1497
Val Pro His Pro Asp Val Lys Pro Met Ala Tyr Ala Asn Asn Phe Leu
                470                 475                 480

cgc atg ttt tct taaaaactct ggaacctaac gttcagtatt acaatcgatg 1549
Arg Met Phe Ser
            485

cgaccttttg tctgactgac caagagcata cacatcagga ggcaaaagag ctagtactta 1609

cagccaatta gctgcagtgt cattattcgg acaacttcct gacttgcccg cagcatgtga 1669

ggatcctcac ctcctttgtt ttgattaagg cgtaaacact ttctctacga taacaatgtc 1729

gtgtggtgga ttgtcctctg tattatttat attcctcttt gtaagattgt tacacttctc 1789

atttctttta gaatgttcaa attgggaaaa gctttgataa gtcgaatttt ggttttttct 1849

aaaaaaaaaa aaaaaaaaaa aaaaaaaaaa aaaaaa 1885

<210> 10
<211> 485
<212> PRT
<213> Lycopersicon esculentum

<400> 10
Met Pro Gly Met Glu Ala Glu Asn Ser Asn Ala Ser Arg Ala Glu Glu
1               5                   10                  15

Leu Lys Gln Leu Ala Asn Glu Ala Phe Lys Gly His Lys Tyr Ser Gln
            20                  25                  30

Ala Ile Asp Leu Tyr Thr Gln Ala Ile Glu Leu Asn Gly Glu Asn Ala
        35                  40                  45

Val Tyr Tyr Ala Asn Arg Ala Phe Ala His Thr Lys Leu Glu Glu Tyr
50                  55                  60

Gly Ser Ala Ile Gln Asp Gly Thr Arg Ala Ile Glu Ile Asp Pro Arg
65                  70                  75                  80

Tyr Ser Lys Gly Tyr Tyr Arg Arg Gly Ala Ala Tyr Leu Ala Met Gly
                85                  90                  95

Lys Phe Lys Asp Ala Leu Lys Asp Phe Gln Gln Val Lys Lys Leu Cys
            100                 105                 110

Pro Asn Asp Pro Asp Ala Thr Lys Lys Leu Lys Glu Cys Glu Lys Ala
        115                 120                 125

Val Met Lys Leu Lys Phe Glu Glu Ala Ile Ser Val Pro Glu Ser Gln
    130                 135                 140

Arg Arg Ser Val Ala Asp Ser Ile Asp Tyr Arg Ser Val Glu Val Glu
145                 150                 155                 160

Pro Gln Tyr Ala Gly Ala Arg Ile Glu Gly Asp Val Val Thr Leu Asp
                165                 170                 175

Phe Val Lys Lys Met Leu Asp Asp Phe Lys Asn Gln Lys Asn Leu His
            180                 185                 190

Lys Arg Tyr Ala Tyr Gln Ile Val Leu Gln Thr Arg Glu Met Leu Arg
        195                 200                 205

Ala Leu Pro Ser Leu Val Asp Ile Val Val Pro Glu Gly Lys His Phe
    210                 215                 220

Thr Val Cys Gly Asp Val His Gly Gln Phe Tyr Asp Leu Leu Asn Ile
225                 230                 235                 240

Phe Glu Leu Asn Gly Leu Pro Ser Glu Asp Asn Pro Tyr Leu Phe Asn
                245                 250                 255

Gly Asp Phe Val Asp Arg Gly Ser Phe Ser Leu Glu Val Ile Leu Thr
            260                 265                 270

Leu Phe Ala Phe Lys Cys Met Cys Pro Ser Ala Ile His Leu Ala Arg
        275                 280                 285

Gly Asn His Glu Ser Lys Ser Met Asn Lys Ile Tyr Gly Phe Glu Gly
    290                 295                 300

Glu Val Arg Ser Lys Leu Ser Glu Ile Phe Val Glu Leu Phe Ala Glu
305                 310                 315                 320

Val Phe Cys Cys Leu Pro Leu Ala His Val Ile Asn Glu Lys Val Phe
                325                 330                 335

Val Val His Arg Gly Leu Phe Ser Val Asp Gly Val Lys Leu Ser Asp
            340                 345                 350

Ile Arg Ala Ile Asp Arg Phe Cys Glu Pro Pro Glu Glu Gly Leu Met
        355                 360                 365

Cys Glu Leu Leu Trp Ser Asp Pro Gln Pro Gln Pro Gly Arg Gly Pro
    370                 375                 380

Ser Lys Arg Gly Val Gly Leu Ser Phe Gly Gly Asp Val Thr Lys Arg
385                 390                 395                 400

Phe Leu Gln Glu Asn Asn Leu Asp Leu Val Val Arg Ser His Glu Val
                405                 410                 415

Lys Asp Glu Gly Tyr Glu Ile Glu His Asp Gly Lys Leu Ile Thr Val
            420                 425                 430

Phe Ser Ala Pro Asn Tyr Cys Asp Gln Met Gly Asn Lys Gly Ala Phe
        435                 440                 445

Ile Arg Phe Glu Ala Pro Asp Met Lys Pro Asn Ile Val Thr Phe Ser
    450                 455                 460

Ala Val Pro His Pro Asp Val Lys Pro Met Ala Tyr Ala Asn Asn Phe
465                 470                 475                 480

Leu Arg Met Phe Ser
                485

<210> 11
<211> 153
<212> PRT
<213> Lycopersicon esculentum

<400> 11
Met Ser Leu Ile Pro Ser Ile Phe Gly Gly Arg Arg Ser Asn Val Phe
  1               5                  10                  15

Asp Pro Phe Ser Leu Asp Val Trp Asp Pro Phe Lys Asp Phe His Phe

20                  25                  30

Pro Thr Ser Leu Ser Ala Glu Asn Ser Ala Phe Val Asn Thr Arg Val
        35                  40                  45

Asp Trp Lys Glu Thr Pro Glu Ala His Val Phe Glu Ala Asp Ile Pro
    50                  55                  60

Gly Leu Lys Lys Glu Glu Val Lys Val Gln Ile Glu Asp Asp Arg Val
65                  70                  75                  80

Leu Gln Ile Ser Gly Glu Arg Asn Leu Glu Lys Glu Asp Lys Asn Asp
                85                  90                  95

Thr Trp His Arg Val Glu Arg Ser Ser Gly Asn Phe Met Arg Arg Phe
            100                 105                 110

Arg Leu Pro Glu Asn Ala Lys Val Glu Gln Val Lys Ala Ser Met Glu
        115                 120                 125

Asn Gly Val Leu Thr Val Thr Val Pro Lys Glu Glu Val Lys Lys Pro
    130                 135                 140

Asp Val Lys Ala Ile Glu Ile Ser Gly
145                 150

<210> 12
<211> 153
<212> PRT
<213> Lycopersicon esculentum

<400> 12
Met Ser Met Ile Pro Ser Phe Phe Asn Asn Asn Arg Arg Ser Asn Ile
1               5                   10                  15

Phe Asp Pro Phe Ser Leu Asp Val Trp Asp Pro Phe Lys Glu Leu Thr
            20                  25                  30

Ser Ser Ser Leu Ser Arg Glu Asn Ser Ala Ile Val Asn Ala Arg Val
        35                  40                  45

Asp Trp Arg Glu Thr Pro Glu Ala His Val Phe Lys Ala Asp Leu Pro
    50                  55                  60

Gly Leu Lys Lys Glu Glu Val Lys Val Glu Ile Glu Glu Asp Ser Val
65                  70                  75                  80

Leu Lys Ile Ser Gly Glu Arg His Val Glu Lys Glu Asp Lys Asn Asp
                85                  90                  95

Thr Trp His Arg Val Glu Arg Ser Ser Gly Gln Phe Thr Arg Arg Phe
            100                 105                 110

Arg Leu Pro Glu Asn Val Lys Met Asp Gln Val Lys Ala Ala Met Glu
        115                 120                 125

Asn Gly Val Leu Thr Val Thr Val Pro Lys Ala Glu Thr Lys Lys Ala
    130                 135                 140

Asp Val Lys Ser Ile Gln Ile Ser Gly
145                 150

<210> 13
<211> 151
<212> PRT
<213> Lycopersicon esculentum

<400> 13
Met Ser Ile Val Arg Arg Ser Asn Val Phe Asp Pro Phe Ala Asp Leu
  1               5                  10                  15

Trp Ala Asp Pro Phe Asp Thr Phe Arg Ser Ile Val Pro Ala Ile Ser
            20                  25                  30

Gly Gly Ser Ser Glu Thr Ala Ala Phe Ala Asn Ala Arg Val Asp Trp
        35                  40                  45

Lys Glu Thr Pro Glu Ala His Val Phe Lys Val Asp Leu Pro Gly Val
    50                  55                  60

Lys Lys Glu Glu Val Lys Val Glu Val Glu Asp Gly Asn Val Leu Val
 65                  70                  75                  80

Val Ser Gly Glu Arg Ser Arg Glu Lys Glu Asp Lys Asn Asp Lys Trp
                85                  90                  95

His Arg Val Glu Arg Ser Ser Gly Lys Phe Val Arg Arg Phe Arg Leu
            100                 105                 110

Pro Glu Asp Ala Lys Val Glu Glu Val Lys Ala Gly Leu Glu Asn Gly
        115                 120                 125

Val Leu Thr Val Thr Val Pro Lys Ala Glu Val Lys Lys Pro Glu Val
    130                 135                 140

Lys Ala Ile Glu Ile Ser Gly
145                 150

<210> 14
<211> 152
<212> PRT
<213> Lycopersicon esculentum

<400> 14
Met Ser Leu Val Arg Arg Ser Asn Val Phe Asp Pro Phe Ser Met Asp
  1               5                  10                  15

Leu Trp Asp Pro Phe Asp Thr Met Phe Arg Ser Ile Val Pro Ser Ala
             20                  25                  30

Thr Ser Thr Asn Ser Glu Thr Ala Ala Phe Ala Ser Ala Arg Ile Asp
         35                  40                  45

Trp Lys Glu Thr Pro Glu Ala His Val Phe Lys Ala Asp Leu Pro Gly
     50                  55                  60

Val Lys Lys Glu Glu Val Lys Val Glu Val Glu Asp Gly Asn Val Leu
 65                  70                  75                  80

Val Ile Ser Gly Gln Arg Ser Arg Glu Lys Glu Asp Lys Asp Asp Lys
                 85                  90                  95

Trp His Arg Val Glu Arg Ser Ser Gly Gln Phe Ile Arg Arg Phe Arg
            100                 105                 110

Leu Pro Asp Asp Ala Lys Val Asp Gln Val Lys Ala Gly Leu Glu Asn
        115                 120                 125

Gly Val Leu Thr Val Thr Val Pro Lys Ala Glu Glu Lys Lys Pro Glu
    130                 135                 140

Val Lys Ala Ile Glu Ile Ser Gly
145                 150

<210> 15
<211> 217

<212> PRT
<213> Lycopersicon esculentum

<400> 15
Ile Arg His Glu Ala Ala Arg Lys Leu Arg Glu Asn Gln Ser Thr Thr
1               5                   10                  15

Ser Val Gln Thr Glu Asp Leu Val Glu Asp Pro Ala Gln Glu Ser Ala
            20                  25                  30

Asp Tyr His Arg Asn Leu Gly Leu Gln Met Val Ser Gly Leu Ser Asn
        35                  40                  45

Glu Leu Glu Asn Val Arg Lys Ala Ser Leu Ile Asp Gly Glu Asn Leu
    50                  55                  60

Ser Ala Ala Val Met Lys Leu Asn His Ser Leu Met Lys Thr Lys Glu
65                  70                  75                  80

Phe Leu Asp Thr Asp Met Arg Ser Leu Glu Asp Glu Ser Lys Phe Arg
                85                  90                  95

Asp Thr Leu Thr Asn Phe Ile Gln His Ala Glu Gln Asp Ile Thr Cys
            100                 105                 110

Ile Leu Glu Glu Glu Lys Lys Ile Met Ser Leu Val Lys Ser Thr Gly
        115                 120                 125

Asp Tyr Phe His Gly Asn Ser Gly Lys Asp Glu Gly Leu Arg Leu Phe
    130                 135                 140

Ser Val Val Ser Asp Phe Leu Ile Met Leu Asp Lys Ala Cys Thr Val
145                 150                 155                 160

Val Arg Asn Ser Thr Lys Leu Pro Val Lys Ile Pro Lys Lys Gly Thr
                165                 170                 175

Leu Thr Ser Pro Ser Gln Glu Ser Cys Pro Glu Ser Leu Gln Asp Ile
            180                 185                 190

Arg Lys Gln Leu Phe Pro Ala Ile Gln Glu Arg Gln Met His Tyr Ser
        195                 200                 205

Ser Ser Asp Asp Glu Ser Ser Ser Pro
    210                 215

<210> 16
<211> 256
<212> PRT
<213> Arabidopsis arenosa

<400> 16
Arg Met Asn Val Gly Thr Phe Arg Gly Asp Ala Gln Ala Phe Lys Leu
1               5                   10                  15

Asp Thr Leu Leu Lys Leu Ser Asp Val Lys Gly Thr Asp Gly Lys Thr
            20                  25                  30

Thr Leu Leu His Phe Val Val Leu Glu Ile Ile Arg Ser Glu Gly Val
        35                  40                  45

Arg Ala Leu Arg Leu Gln Ser Arg Ser Phe Ser Ser Val Lys Thr Asp
    50                  55                  60

Asp Ser Asn Ala Asp Ser Ser Pro Gln Ser Val Asp Tyr Arg Ser Thr
65                  70                  75                  80

Gly Leu Gln Val Val Thr Gly Leu Thr Thr Glu Leu Glu Asp Val Lys
                85                  90                  95

Arg Ala Ala Ile Ile Asp Ala Asp Gly Leu Ala Ala Thr Leu Ala Asn
            100                 105                 110

Ile Ser Gly Ser Leu Thr Asn Ala Arg Glu Phe Leu Lys Thr Met Asp
        115                 120                 125

Glu Glu Ser Asp Phe Glu Arg Ala Leu Ala Gly Phe Ile Glu Arg Ala
    130                 135                 140

Asp Ala Asp Phe Lys Trp Leu Lys Glu Glu Glu Glu Arg Ile Met Val
145                 150                 155                 160

Leu Val Lys Ser Ser Ala Asp Tyr Phe His Gly Lys Ser Ala Lys Asn
                165                 170                 175

Glu Gly Leu Arg Leu Phe Ala Ile Val Arg Asp Phe Leu Ile Met Leu
            180                 185                 190

Glu Lys Val Cys Arg Glu Val Lys Glu Thr Thr Lys Thr Thr Asn His
        195                 200                 205

Ser Gly Lys Lys Glu Ser Glu Met Thr Thr Ser Asp Ser Asn Gln Pro
    210                 215                 220

Ser Pro Asp Phe Arg Gln Arg Leu Phe Pro Ala Ile Ala Glu Arg Arg
225                 230                 235                 240

Met Asp Ser Ser Asp Asp Ser Asp Asp Ser Glu Glu Asp Ser Ser Pro
                245                 250                 255

<210> 17
<211> 242
<212> PRT
<213> Arabidopsis thaliana

<400> 17
Arg Met Asn Val Gly Thr Asn Arg Gly Asp Ala His Ala Phe Lys Leu
  1               5                  10                  15

Asp Thr Leu Leu Lys Leu Val Asp Val Lys Gly Ala Asp Gly Lys Thr
             20                  25                  30

Thr Leu Leu His Phe Val Val Gln Glu Ile Ile Arg Ala Glu Gly Thr
         35                  40                  45

Arg Leu Ser Gly Asn Asn Thr Gln Thr Asp Asp Ile Lys Cys Arg Lys
     50                  55                  60

Leu Gly Leu Gln Val Val Ser Ser Leu Cys Ser Glu Leu Ser Asn Val
 65                  70                  75                  80

Lys Lys Ala Ala Ala Met Asp Ser Glu Val Leu Ser Ser Tyr Val Ser
                 85                  90                  95

Lys Leu Ser Gln Gly Ile Ala Lys Ile Asn Glu Ala Ile Gln Val Gln
            100                 105                 110

Ser Thr Ile Thr Glu Glu Ser Asn Ser Gln Arg Phe Ser Glu Ser Met
        115                 120                 125

Lys Thr Phe Leu Lys Arg Ala Glu Glu Glu Ile Ile Arg Val Gln Ala
    130                 135                 140

Gln Glu Ser Val Ala Leu Ser Leu Val Lys Glu Ile Thr Glu Tyr Phe
145                 150                 155                 160

His Gly Asn Ser Ala Lys Glu Glu Ala His Pro Phe Arg Ile Phe Leu

                165                 170                 175

Val Val Arg Asp Phe Leu Gly Val Val Asp Arg Val Cys Lys Glu Val
            180                 185                 190

Gly Met Ile Asn Glu Arg Thr Met Val Ser Ser Ala His Lys Phe Pro
        195                 200                 205

Val Pro Val Asn Pro Met Met Pro Gln Pro Leu Pro Gly Leu Val Gly
    210                 215                 220

Arg Arg Gln Ser Ser Ser Ser Ser Ser Ser Ser Ser Thr Ser Ser Ser
225                 230                 235                 240

Asp Glu

<210> 18
<211> 245
<212> PRT
<213> Nicotiana tabacum

<400> 18
Arg Met Asn Val Gly Thr Asn Arg Gly Asp Ala His Ala Phe Lys Leu
1               5                   10                  15

Asp Thr Leu Leu Lys Leu Val Asp Val Lys Gly Ala Asp Gly Lys Thr
            20                  25                  30

Thr Leu Leu His Phe Val Val Gln Glu Ile Ile Lys Ser Glu Gly Ala
        35                  40                  45

Arg Leu Ser Gly Gly Asn Gln Asn His Gln Gln Ser Thr Thr Asn Asp
    50                  55                  60

Asp Ala Lys Cys Lys Lys Leu Gly Leu Gln Val Val Ser Asn Ile Ser
65                  70                  75                  80

Ser Glu Leu Ile Asn Val Lys Lys Ser Ala Ala Met Asp Ser Glu Val
                85                  90                  95

Leu His Asn Asp Val Leu Lys Leu Ser Lys Gly Ile Gln Asn Ile Ala
            100                 105                 110

Glu Val Val Arg Ser Ile Glu Ala Val Gly Leu Glu Glu Ser Ser Ile
        115                 120                 125

Lys Arg Phe Ser Glu Ser Met Asn Arg Phe Met Lys Val Ala Glu Glu
    130                 135                 140

Lys Ile Leu Arg Leu Gln Ala Gln Glu Thr Leu Ala Met Ser Leu Val
145                 150                 155                 160

Lys Glu Ile Thr Glu Tyr Val His Gly Asp Ser Ala Arg Glu Glu Ala
                165                 170                 175

His Pro Phe Arg Ile Phe Met Val Val Lys Asp Phe Leu Met Ile Leu
            180                 185                 190

Asp Cys Val Cys Lys Glu Val Gly Thr Ile Asn Glu Arg Thr Ile Val
        195                 200                 205

Ser Ser Ala Gln Lys Phe Pro Val Pro Val Asn Pro Asn Leu Gln Pro
    210                 215                 220

Val Ile Ser Gly Phe Arg Ala Lys Arg Leu His Ser Ser Ser Asp Glu
225                 230                 235                 240

Glu Ser Ser Ser Pro
                245

<210> 19
<211> 117
<212> PRT
<213> Picea mariana

<400> 19
Gln Gly Asn Phe Phe Lys Ser Met Ser Ser Phe Leu Gln Glu Ala Glu
1               5                   10                  15

Glu Asp Ile Ala Arg Ile Gln Ser Glu Glu Asn Arg Ala Phe Ser Leu
            20                  25                  30

Val Arg Glu Thr Thr Glu Tyr Phe His Gly Asp Ala Ala Lys Glu Glu
        35                  40                  45

Gly Arg Pro Leu Arg Phe Phe Val Val Val Lys Asp Phe Leu Gly Val
    50                  55                  60

Leu Asp Gln Val Cys Arg Glu Ile Gly Lys Thr Arg Thr Arg Met Ala
65                  70                  75                  80

Gln Ser Ser Pro Arg Pro Pro Gln Val Val Ala His Ala Ile Ser Met
                85                  90                  95

Pro Leu Phe Pro Lys Ala Leu Gln Arg Arg Pro Asp Ser Ser Asp Asp
            100                 105                 110

Glu Ser Ser Ser Pro
        115

<210> 20
<211> 180
<212> PRT
<213> Arabidopsis thaliana

<400> 20
Met Lys Ser Gly Asn Ala His Asp Phe Lys Leu Glu Ala Leu Leu Gly
  1               5                  10                  15

Leu Val Asp Ile Lys Ser Ser Asp Gly Arg Thr Ser Ile Leu Asp Ser
            20                  25                  30

Val Val Gln Lys Ile Thr Glu Ser Glu Gly Ile Lys Gly Leu Gln Val
        35                  40                  45

Val Arg Asn Leu Ser Ser Val Leu Asn Asp Ala Lys Lys Ser Ala Glu
    50                  55                  60

Leu Asp Tyr Gly Val Val Arg Met Asn Val Ser Lys Leu Tyr Glu Glu
 65                  70                  75                  80

Val Gln Lys Ile Ser Glu Val Leu Arg Leu Cys Glu Glu Thr Gly His
                85                  90                  95

Ser Glu Glu His Gln Trp Trp Lys Phe Arg Glu Ser Val Thr Arg Phe
            100                 105                 110

Leu Glu Thr Ala Ala Glu Glu Ile Lys Lys Ile Glu Arg Glu Glu Gly
        115                 120                 125

Ser Thr Leu Phe Ala Val Lys Lys Ile Thr Glu Tyr Phe His Val Asp
    130                 135                 140

Pro Ala Lys Glu Glu Ala Gln Leu Leu Lys Val Phe Val Ile Val Arg
145                 150                 155                 160

Asp Phe Leu Lys Ile Leu Glu Gly Val Cys Lys Lys Met Glu Val Thr

                165                 170                 175

Ser Ser Leu Ala
            180

<210> 21
<211> 98
<212> PRT
<213> Lycopersicon esculentum

<400> 21
Ala Asp Leu Thr Gly Glu Leu Met Arg Leu Ala Ile Gly Arg Ile Ser
1               5                   10                  15

Glu Gly Glu Leu Asp Phe Ala Glu Lys Ile Cys Ser Phe Ala Arg Glu
            20                  25                  30

Ile Tyr Arg Asn Leu Thr Leu Ile Ala Pro Glu Met Asp Asp Ser Ser
        35                  40                  45

Asp Met Lys Gln Lys Met Glu Thr Met Leu Gln Ser Val Met Lys Ile
    50                  55                  60

Glu Asn Ala Cys Phe Ser Val His Val Arg Gly Ser Glu Tyr Ile Pro
65                  70                  75                  80

Leu Leu Gly Pro Ala Asp Thr Ser Tyr Pro Leu Leu Gly Met Pro Asp
                85                  90                  95

Ile Glu

<210> 22
<211> 299
<212> PRT
<213> Arabidopsis thaliana

<400> 22
Met Leu Ser Cys Ser Ser Ser Ala Phe Gln Arg Val Ala Phe Met Leu
1               5                   10                  15

Met Ala Pro Lys Leu Lys Pro Gln Arg Leu His Gln Met Leu Ile Ser
            20                  25                  30

Asn Asp Gly Phe Gly Val Cys Val Val Ala Glu Ser Gly Val Glu His
        35                  40                  45

Leu Val Lys Lys Ala Arg Thr Met Ser Thr Glu Ser Ser Met Lys Asp
    50                  55                  60

Ala Phe Ser Thr Tyr Ala Asp Tyr Leu Asn Asn Phe Asn Glu Lys Arg
65                  70                  75                  80

Glu Arg Val Val Lys Val Ser Arg Asp Ile Thr Met Asn Ser Lys Lys
                85                  90                  95

Val Ile Phe Gln Val His Arg Leu Ser Lys Asp Asn Lys Glu Glu Val
            100                 105                 110

Leu Glu Lys Ala Gly Lys Asp Leu Glu Ala Val Arg Asp Gln His Phe
        115                 120                 125

Ala Arg Leu Met Lys Glu Leu Gln Gly Thr Asp Phe Trp Lys Leu Arg
    130                 135                 140

Arg Ala Tyr Ser Pro Gly Val Gln Glu Tyr Val Glu Ala Ala Thr Phe
145                 150                 155                 160

Tyr Lys Phe Cys Leu Ser Gly Thr Leu Cys Thr Leu Asp Glu Ile Asn
                165                 170                 175

Thr Thr Leu Val Pro Leu Ser Asp Pro Ser Leu Glu Pro Leu Gln Ile
            180                 185                 190

Asn Ile Leu Asp Tyr Ile Leu Gly Leu Ala Asp Leu Thr Gly Glu Leu
        195                 200                 205

Met Arg Met Ala Ile Gly Arg Ile Ser Asp Gly Glu Ile Glu Phe Ala
    210                 215                 220

Gln Arg Ile Cys Gln Phe Val Arg Gln Ile His Arg Glu Leu Met Leu
225                 230                 235                 240

Val Val Pro Lys Met Asp Asp Ser Tyr Asp Met Lys Ser Lys Met Glu
                245                 250                 255

Val Met Leu Gln Ser Val Ile Lys Ile Glu Asn Ala Cys Phe Ser Val
            260                 265                 270

His Val Arg Gly Leu Glu Tyr Ile Pro Leu Leu Gly Asp Asn Ala Pro
        275                 280                 285

Thr Ser Tyr Leu Leu Gly Ala Ala Asp Val Glu
    290                 295

<210> 23
<211> 300
<212> PRT
<213> Oryza sativa

<400> 23
Met Leu Pro Leu Arg Gly Cys His Arg Arg Leu Leu Ser Leu Arg Gly
1               5                   10                  15

Val Thr Ala Pro Ser Leu Leu Pro Pro Ile Thr Thr Thr Pro Thr Thr
            20                  25                  30

Ser Met Ala Ala Pro Gln Ser His Ser His Pro Ala Lys Thr Leu Arg
        35                  40                  45

Ala Ser Pro Pro Pro Pro Ser Thr Ala Gly Ser Ala Pro Lys Arg Ser
    50                  55                  60

Arg Thr Met Ala Thr Asp Ala Ala Ala Thr Ala His Ser Ala Ser Ala
65                  70                  75                  80

Gly Cys Ser Ala Met Lys Ala Glu Phe Ala Lys His Ala Glu Tyr Leu
                85                  90                  95

Asn Thr Leu Asn Asp Lys Arg Glu Arg Leu Val Lys Ala Ser Arg Asp
            100                 105                 110

Leu Thr Met Asn Ser Lys Lys Ala Ile Phe Gln Val His Arg Ile Ser
        115                 120                 125

Lys Asn Asn Lys Glu Glu Val Leu Ser Lys Ala Glu Asn Asp Leu Thr
    130                 135                 140

Val Val Val Asn Gln Tyr Ile Gly Lys Leu Val Lys Glu Leu Gln Gly
145                 150                 155                 160

Thr Asp Phe Trp Lys Leu Arg Arg Ala Tyr Thr Phe Gly Val Gln Glu
                165                 170                 175

Tyr Val Glu Ala Ala Thr Phe Cys Arg Phe Cys Lys Thr Gly Thr Leu
            180                 185                 190

Leu Ser Leu Ala Glu Ile Asn Asp Ser Leu Leu Glu Leu Gly Asp Lys

195                 200                 205

Ser Val Glu Pro Leu Gln Ile Asn Val Leu Asp Tyr Val Leu Gly Val
    210                 215                 220

Ala Asp Leu Ser Gly Glu Leu Met Arg Leu Ala Ile Gly Arg Ile Ser
225                 230                 235                 240

Asp Gly Glu Val Glu Tyr Ala Lys Asn Ile Cys Ala Phe Val Arg Asp
                245                 250                 255

Ile Tyr Arg Glu Leu Thr Leu Val Val Pro Leu Met Asp Asp Asn Ser
            260                 265                 270

Glu Met Lys Lys Lys Met Glu Thr Met Leu Gln Ser Val Val Lys Ile
        275                 280                 285

Glu Asn Ala Cys Phe Ser Val His Val Arg Gly Ser
    290                 295                 300

<210> 24
<211> 93
<212> PRT
<213> Lycopersicon esculentum

<400> 24
Ile Arg His Glu Leu Ala Ala Thr Tyr Asp Ala Ile Gly Arg Val Asp
  1               5                  10                  15

Asp Ala Ile Glu Ile Leu Glu Tyr Val Leu Lys Leu Arg Glu Glu Lys
             20                  25                  30

Leu Gly Thr Ala Asn Pro Asp Phe Asn Asp Glu Lys Lys Arg Leu Ala
         35                  40                  45

Glu Leu Leu Lys Glu Ala Gly Arg Ser Arg Asn Lys Asn Pro Asn Ser
     50                  55                  60

Leu Glu Asn Leu Ile Asp Pro Asn Ser Lys Arg Thr Thr Lys Lys Glu
 65                  70                  75                  80

Thr Ser Ser Lys Lys Trp Ser Ala Phe Gly Phe Arg Ser
                 85                  90

<210> 25
<211> 248
<212> PRT
<213> Arabidopsis thaliana

<400> 25
Thr Gly Lys Leu Arg Glu Ser Lys Ser Tyr Cys Glu Asn Ala Leu Arg
1               5                   10                  15

Ile Tyr Asn Lys Pro Val Pro Gly Thr Thr Val Glu Glu Ile Ala Gly
            20                  25                  30

Gly Leu Thr Glu Ile Ser Ala Ile Tyr Glu Ser Val Asp Glu Pro Glu
        35                  40                  45

Glu Ala Leu Lys Leu Leu Gln Lys Ser Met Lys Leu Leu Glu Asp Lys
    50                  55                  60

Pro Gly Gln Gln Ser Ala Ile Ala Gly Leu Glu Ala Arg Met Gly Val
65                  70                  75                  80

Met Tyr Tyr Thr Val Gly Arg Tyr Glu Asp Ala Arg Asn Ala Phe Glu
                85                  90                  95

Ser Ala Val Thr Lys Leu Arg Ala Ala Gly Glu Lys Ser Ala Phe Phe
            100                 105                 110

Gly Val Val Leu Asn Gln Met Gly Leu Ala Cys Val Gln Leu Phe Lys
        115                 120                 125

Ile Asp Glu Ala Gly Glu Leu Phe Glu Glu Ala Arg Gly Ile Leu Glu
    130                 135                 140

Gln Glu Arg Gly Pro Cys Asp Gln Asp Thr Leu Gly Val Tyr Ser Asn
145                 150                 155                 160

Leu Ala Ala Thr Tyr Asp Ala Met Gly Arg Ile Glu Asp Ala Ile Glu
                165                 170                 175

Ile Leu Glu Gln Val Leu Lys Leu Arg Glu Glu Lys Leu Gly Thr Ala
            180                 185                 190

Asn Pro Asp Phe Glu Asp Glu Lys Lys Arg Leu Ala Glu Leu Leu Lys
        195                 200                 205

Glu Ala Gly Arg Ser Arg Asn Tyr Lys Ala Lys Ser Leu Gln Asn Leu
    210                 215                 220

Ile Asp Pro Asn Ala Arg Pro Pro Lys Lys Glu Ser Ser Ala Lys Lys
225                 230                 235                 240

Trp Pro Ser Leu Gly Phe Lys Phe
                245

<210> 26
<211> 220
<212> PRT
<213> Arabidopsis thaliana

<400> 26
Thr Gly Lys Val Arg Glu Ala Lys Ser Tyr Cys Glu Asn Ala Leu Arg
  1               5                  10                  15

Ile Tyr Glu Ser His Asn Leu Glu Ile Ser Pro Glu Glu Ile Ala Ser
             20                  25                  30

Gly Leu Thr Asp Ile Ser Val Ile Cys Glu Ser Met Asn Glu Val Glu
         35                  40                  45

Gln Ala Ile Thr Leu Leu Gln Lys Ala Leu Lys Ile Tyr Ala Asp Ser
     50                  55                  60

Pro Gly Gln Lys Ile Met Ile Ala Gly Ile Glu Ala Gln Met Gly Val
 65                  70                  75                  80

Leu Tyr Tyr Met Met Gly Lys Tyr Met Glu Ser Tyr Asn Thr Phe Lys
                 85                  90                  95

Ser Ala Ile Ser Lys Leu Arg Ala Thr Gly Lys Lys Gln Ser Thr Phe
            100                 105                 110

Phe Gly Ile Ala Leu Asn Gln Met Gly Leu Ala Cys Ile Gln Leu Asp
        115                 120                 125

Ala Ile Glu Glu Ala Val Glu Leu Phe Glu Glu Ala Lys Cys Ile Leu
    130                 135                 140

Glu Gln Glu Cys Gly Pro Tyr His Pro Glu Thr Leu Gly Leu Tyr Ser
145                 150                 155                 160

Asn Leu Ala Gly Ala Tyr Asp Ala Ile Gly Arg Leu Asp Asp Ala Ile
                165                 170                 175

Lys Leu Leu Gly His Val Val Gly Val Arg Glu Glu Lys Leu Gly Thr

180                 185                 190

Ala Asn Pro Val Thr Glu Asp Glu Lys Arg Arg Leu Ala Gln Leu Leu
        195                 200                 205

Lys Glu Ala Gly Asn Val Thr Gly Arg Lys Ala Lys
    210                 215                 220

<210> 27
<211> 482
<212> PRT
<213> Lycopersicon esculentum

<400> 27
Met Glu Ala Glu Asn Ser Asn Ala Ser Arg Ala Glu Glu Leu Lys Gln
1               5                   10                  15

Leu Ala Asn Glu Ala Phe Lys Gly His Lys Tyr Ser Gln Ala Ile Asp
            20                  25                  30

Leu Tyr Thr Gln Ala Ile Glu Leu Asn Gly Glu Asn Ala Val Tyr Tyr
        35                  40                  45

Ala Asn Arg Ala Phe Ala His Thr Lys Leu Glu Glu Tyr Gly Ser Ala
    50                  55                  60

Ile Gln Asp Gly Thr Arg Ala Ile Glu Ile Asp Pro Arg Tyr Ser Lys
65                  70                  75                  80

Gly Tyr Tyr Arg Arg Gly Ala Ala Tyr Leu Ala Met Gly Lys Phe Lys
                85                  90                  95

Asp Ala Leu Lys Asp Phe Gln Gln Val Lys Lys Leu Cys Pro Asn Asp
            100                 105                 110

Pro Asp Ala Thr Lys Lys Leu Lys Glu Cys Glu Lys Ala Val Met Lys
        115                 120                 125

Leu Lys Phe Glu Glu Ala Ile Ser Val Pro Glu Ser Gln Arg Arg Ser
    130                 135                 140

Val Ala Asp Ser Ile Asp Tyr Arg Ser Val Glu Val Glu Pro Gln Tyr
145                 150                 155                 160

Ala Gly Ala Arg Ile Glu Gly Asp Val Val Thr Leu Asp Phe Val Lys
                165                 170                 175

Lys Met Leu Asp Asp Phe Lys Asn Gln Lys Asn Leu His Lys Arg Tyr
            180                 185                 190

Ala Tyr Gln Ile Val Leu Gln Thr Arg Glu Met Leu Arg Ala Leu Pro
        195                 200                 205

Ser Leu Val Asp Ile Val Val Pro Glu Gly Lys His Phe Thr Val Cys
    210                 215                 220

Gly Asp Val His Gly Gln Phe Tyr Asp Leu Leu Asn Ile Phe Glu Leu
225                 230                 235                 240

Asn Gly Leu Pro Ser Glu Asp Asn Pro Tyr Leu Phe Asn Gly Asp Phe
                245                 250                 255

Val Asp Arg Gly Ser Phe Ser Leu Glu Val Ile Leu Thr Leu Phe Ala
            260                 265                 270

Phe Lys Cys Met Cys Pro Ser Ala Ile His Leu Ala Arg Gly Asn His
        275                 280                 285

Glu Ser Lys Ser Met Asn Lys Ile Tyr Gly Phe Glu Gly Glu Val Arg
    290                 295                 300

Ser Lys Leu Ser Glu Ile Phe Val Glu Leu Phe Ala Glu Val Phe Cys
305                 310                 315                 320

Cys Leu Pro Leu Ala His Val Ile Asn Glu Lys Val Phe Val Val His
                325                 330                 335

Arg Gly Leu Phe Ser Val Asp Gly Val Lys Leu Ser Asp Ile Arg Ala
            340                 345                 350

Ile Asp Arg Phe Cys Glu Pro Pro Glu Glu Gly Leu Met Cys Glu Leu
        355                 360                 365

Leu Trp Ser Asp Pro Gln Pro Gln Pro Gly Arg Gly Pro Ser Lys Arg
    370                 375                 380

Gly Val Gly Leu Ser Phe Gly Gly Asp Val Thr Lys Arg Phe Leu Gln
385                 390                 395                 400

Glu Asn Asn Leu Asp Leu Val Val Arg Ser His Glu Val Lys Asp Glu
                405                 410                 415

Gly Tyr Glu Ile Glu His Asp Gly Lys Leu Ile Thr Val Phe Ser Ala
            420                 425                 430

Pro Asn Tyr Cys Asp Gln Met Gly Asn Lys Gly Ala Phe Ile Arg Phe
        435                 440                 445

Glu Ala Pro Asp Met Lys Pro Asn Ile Val Thr Phe Ser Ala Val Pro
    450                 455                 460

His Pro Asp Val Lys Pro Met Ala Tyr Ala Asn Asn Phe Leu Arg Met
465                 470                 475                 480

Phe Ser

<210> 28
<211> 533
<212> PRT
<213> Arabidopsis thaliana

<400> 28
Met Glu Thr Lys Asn Glu Asn Ser Asp Val Ser Arg Ala Glu Glu Phe
  1               5                  10                  15

Lys Ser Gln Ala Asn Glu Ala Phe Lys Gly His Lys Tyr Ser Ser Ala
             20                  25                  30

Ile Asp Leu Tyr Thr Lys Ala Ile Glu Leu Asn Ser Asn Asn Ala Val
         35                  40                  45

Tyr Trp Ala Asn Arg Ala Phe Ala His Thr Lys Leu Glu Glu Tyr Gly
     50                  55                  60

Ser Ala Ile Gln Asp Ala Ser Lys Ala Ile Glu Val Asp Ser Arg Tyr
 65                  70                  75                  80

Ser Lys Gly Tyr Tyr Arg Arg Gly Ala Ala Tyr Leu Ala Met Gly Lys
                 85                  90                  95

Phe Lys Asp Ala Leu Lys Asp Phe Gln Gln Val Lys Arg Leu Ser Pro
            100                 105                 110

Asn Asp Pro Asp Ala Thr Arg Lys Leu Lys Glu Cys Glu Lys Ala Val
        115                 120                 125

Met Lys Leu Lys Phe Glu Glu Ala Ile Ser Val Pro Val Ser Glu Arg
    130                 135                 140

Arg Ser Val Ala Glu Ser Ile Asp Phe His Thr Ile Gly Asn Lys Pro
145                 150                 155                 160

Arg Ser Ser Ser Met Pro Thr Lys Thr Ala Leu Ala Ala Val Val Ala
                165                 170                 175

Ala Val Met Val Val Ala Val Arg Gly Phe Ala Thr Thr Glu Ile Leu
            180                 185                 190

Met Val Leu Val Ser Val Val Leu Gly Thr Phe Trp Trp Glu Val Glu
        195                 200                 205

Pro Gln Tyr Ser Gly Ala Arg Ile Glu Gly Glu Glu Val Thr Leu Asp
    210                 215                 220

Phe Val Lys Thr Met Met Glu Asp Phe Lys Asn Gln Lys Thr Leu His
225                 230                 235                 240

Lys Arg Tyr Ala Tyr Gln Ile Val Leu Gln Thr Arg Gln Ile Leu Leu
                245                 250                 255

Ala Leu Pro Ser Leu Val Asp Ile Ser Val Pro His Gly Lys His Ile
            260                 265                 270

Thr Val Cys Gly Asp Val His Gly Gln Phe Tyr Asp Leu Leu Asn Ile
        275                 280                 285

Phe Glu Leu Asn Gly Leu Pro Ser Glu Glu Asn Pro Tyr Leu Phe Asn
    290                 295                 300

Gly Asp Phe Val Asp Arg Gly Ser Phe Ser Val Glu Ile Ile Leu Thr
305                 310                 315                 320

Leu Phe Ala Phe Lys Cys Met Cys Pro Ser Ser Ile Tyr Leu Ala Arg
                325                 330                 335

Gly Asn His Glu Ser Lys Ser Met Asn Lys Ile Tyr Gly Phe Glu Gly
            340                 345                 350

Glu Val Arg Ser Lys Leu Ser Glu Lys Phe Val Asp Leu Phe Ala Glu
        355                 360                 365

Val Phe Cys Tyr Leu Pro Leu Ala His Val Ile Asn Gly Lys Val Phe
    370                 375                 380

Val Val His Gly Gly Leu Phe Ser Val Asp Gly Val Lys Leu Ser Asp
385                 390                 395                 400

Ile Arg Ala Ile Asp Arg Phe Cys Glu Pro Pro Glu Glu Gly Leu Met
                405                 410                 415

Cys Glu Leu Leu Trp Ser Asp Pro Gln Pro Leu Pro Gly Arg Gly Pro
            420                 425                 430

Ser Lys Arg Gly Val Gly Leu Ser Phe Gly Gly Asp Val Thr Lys Arg
        435                 440                 445

Phe Leu Gln Asp Asn Asn Leu Asp Leu Leu Val Arg Ser His Glu Val
    450                 455                 460

Lys Asp Glu Gly Tyr Glu Val Glu His Asp Gly Lys Leu Ile Thr Val
465                 470                 475                 480

Phe Ser Ala Pro Asn Tyr Cys Asp Gln Met Gly Asn Lys Gly Ala Phe
                485                 490                 495

Ile Arg Phe Glu Ala Pro Asp Met Lys Pro Asn Ile Val Thr Phe Ser
            500                 505                 510

Ala Val Pro His Pro Asp Val Lys Pro Met Ala Tyr Ala Asn Asn Phe
        515                 520                 525

Leu Arg Met Phe Asn
    530